# EUROPEAN PATENT APPLICATION

(11) **EP 2 764 868 A1**
(43) Date of publication of application: **13.08.2014**
(21) Application number: 13154594.9
(22) Date of filing: 08.02.2013
(51) Int. Cl.: A61K 33/04, A61K 33/26, A61P 9/00

(54) **Combination of nitroprusside and a sulfide salt as an HNO-releasing therapeutic for the treatment or prevention of cardiovascular diseases**

(71) Applicant: Friedrich-Alexander-Universität Erlangen-Nürnberg, 91054 Erlangen (DE)
(72) Inventor: Ivanovic-Burmazovic, Ivana, 91080 Weiher/Uttenreuth (DE); Filipovic, Milos, 90403 Nürnberg (DE)
(74) Representative: Vossius & Partner

(57) **Abstract**

The present invention relates to the combination of a nitroprusside salt or a solvate thereof with a sulfide salt or a solvate thereof for use in the treatment or prevention of a cardiovascular disease/disorder, such as heart failure, heart attack or hypertension.

## Description

The present invention relates to the combination of a nitroprusside salt or a solvate thereof with a sulfide salt or a solvate thereof for use in the treatment or prevention of a cardiovascular disease/disorder, such as heart failure, heart attack or hypertension.

Sodium nitroprusside (SNP), i.e. Na₂[Fe(CN)₅NO], is a potent vasodilator, the principal pharmacological action of which is relaxation of vascular smooth muscle and consequent dilatation of peripheral arteries and veins, whereby it is more active on veins than on arteries. Its vasodilatory effects are assigned to the release of nitric oxide NO (referred to as NO in the following). In the human heart, NO reduces both total peripheral resistance as well as venous return, thus decreasing both preload and afterload. SNP is indicated for the immediate reduction of blood pressure of patients in hypertensive crises as well as for the treatment of acute congestive heart failure (package insert for Nitropress® (sodium nitroprusside injection), Hospira, Inc., 2006). However, when SNP is metabolized, it gives rise to considerable quantities of cyanide ions (CN⁻) that can reach toxic, potentially lethal levels, which severely restricts the potential to use SNP as a therapeutic agent. Yet, in certain clinical cases, SNP is still a therapeutic of choice in acute hypertensive crisis, despite its toxicity due to cyanide release.

It would be highly desirable to provide an SNP-based therapeutic composition for the treatment of cardiovascular diseases/disorders, which should not suffer from the disadvantage of causing severe side effects due to the release of toxic cyanide ions from SNP. Accordingly, it is an object of the present invention to provide a novel therapeutic for the medical intervention of cardiovascular diseases/disorders, which should overcome the toxic side effects of SNP resulting from the release of cyanide ions when SNP is used alone.

In the context of the present invention, it has surprisingly been found that, when a nitroprusside salt and a sulfide salt (such as SNP and Na₂S) are used in combination, all of the toxic cyanide that would otherwise be released from nitroprusside is transformed into harmless thiocyanate (SCN⁻), which indicates that the combination of a nitroprusside salt and a sulfide salt lacks the toxicity of nitroprusside alone. Consequently, the combination of a nitroprusside salt and a sulfide salt can be administered safely in higher doses than SNP alone, which further expands the therapeutic potential of this combination.

Hydrogen sulfide (H₂S) has been increasingly recognized as an important signaling molecule. Evidence accumulated over the past decade shows that H₂S plays an important role in regulating vascular contractility and neuronal activity (Li, L. et al., 2009; Li, L. et al., 2011). Experiments using knockout mice lacking cystathionine gamma-lyase, an enzyme involved in H₂S biosynthesis, unambiguously demonstrated its essential role in blood pressure regulation (Yang, G. et al, 2008; Mustafa, A.K. et al., 2011). A strong pharmacological potential for both H₂S and newly developed H₂S donors to minimize the effects of ischemia-reperfusion injuries and atherosclerosis has been documented using numerous disease models (Szabo, C., 2007).

The similarities between the physiological effects of H₂S and NO suggest a possible cross-talk between these two gaseous species (Li, L. et al., 2009). The first such proposal came from Ali et al. who showed that H₂S inhibits the relaxation of isolated aorta rings induced by sodium nitroprusside (Ali, M.Y. et al., 2006). They proposed the intermediate formation of a stable S-nitrosothiol (RSNO). More recently, Yong et al. suggested that the product of NO and H₂S interactions should be a thiol-modifying agent with a positive inotropic effect on the heart (Yong, Q.C. et al., 2011). In a prior study, they had found similarities between this product and nitroxyl (HNO) (Yong, Q.C. et al., 2010).

In all of these studies, SNP was used as the NO donor. However, SNP is not a true NO donor unless it is exposed to light, and its "NO-like" physiological effects are still not well understood (Butler, A. R. et al., 2002). In SNP, i.e. Na₂[Fe(CN)₅(NO)], NO coordinated to iron has an NO⁺ character and undergoes chemistry that is best described as direct nitrosation rather than NO biochemistry (Roncaroli, F. et al., 2007). The reactions of SNP with low molecular weight thiols, like cysteine and glutathione, have been investigated (Butler, A. R. et al., 1988; Grossi, L. et al., 2005; Johnson, M. D. et al., 1983; Szacilowski, K. et al., 2002), and the intermediate formation of S-nitrosothiol was postulated based on these biomimetic studies to account for the vasodilatory effects of SNP. Studies on the reaction of SNP with H₂S were performed under anaerobic and very alkaline conditions (Butler, A. R. et al., 1988; Rock, P. A. et al., 1965; Quiroga, S.L. et al., 2011), but nothing has definitively been known about the products of this reaction under physiological conditions (Filipovic, M. R. et al., 2012, a). Moreover, none of these studies provide any suggestion how the cyanide toxicity of SNP could possibly be overcome.

In other studies, SNP was employed as an NO donor together with an H₂S donor, and their vasodilatory effects were tested (Hosoki, R. et al., 1997; Wang, Y.F. et al., 2008). However, these studies fail to address the problem of overcoming the toxicity of SNP resulting from the release of cyanide ions and furthermore fail to provide any insights into the molecular interaction of SNP with H₂S. The therapeutic use of SNP is also described, e.g., in WO 2010/015260.

Nitroxyl (HNO), the one electron reduced sibling of NO, is even more potent in regulating blood pressure due to the fact that it stimulates the release of calcitonin gene-related peptide (CGRP) which is the strongest known vasodilator (Wink, D.A. et al., 2003). Additionally, HNO has a positive inotropic effect on the heart (Paolocci, N. et al., 2001; Paolocci, N. et al., 2007) and is considered to be important in saving failing heart and to be a very promising therapeutic agent for cardiovascular diseases (Flores-Santana, W. et al., 2011; Irvine, J.C. et al., 2008). A current goal of pharmaceutical companies is the generation of efficient HNO donors that could be used for the regulation of blood pressure and the treatment of heart failure (DuMond, J.F. et al., 2011; Shoman, M.E. et al., 2011; Keefer, L.K., 2011), particularly because, unlike NO donors, HNO does not cause nitrate-tolerance which is a serious drawback of therapy with NO (Bullen, M.L. et al., 2011). Known HNO donors and their use in the treatment of heart failure and other conditions are described, e.g., in US 2004/0039063, US 2005/0192254, US 2007/0299107, US 2009/0163487 and US 2012/0201907.

As explained above, the present invention addresses the problem of providing an SNP-based therapeutic for the medical intervention of cardiovascular diseases/disorders, which should not entail the severe side effects resulting from toxic cyanide ions released from SNP. The invention is based on the finding that, when a nitroprusside salt and a sulfide salt are used in combination, the toxic cyanide that would otherwise be released from the nitroprusside is transformed into harmless thiocyanate (SCN⁻), which was completely unexpected in view of the prior art.

Accordingly, the present invention provides a nitroprusside salt or a solvate thereof in combination with a sulfide salt or a solvate thereof for use in the treatment or prevention of a cardiovascular disease/disorder.

The invention also relates to a pharmaceutical composition for use in the treatment or prevention of a cardiovascular disease/disorder, the pharmaceutical composition comprising a nitroprusside salt or a solvate thereof in combination with a sulfide salt or a solvate thereof, and optionally a pharmaceutically acceptable excipient. Likewise, the present invention relates to the use of a nitroprusside salt or a solvate thereof in combination with a sulfide salt or a solvate thereof for the preparation of a medicament for the treatment or prevention of a cardiovascular disease/disorder.

The invention further provides a nitroprusside salt or a solvate thereof for use in the treatment or prevention of a cardiovascular disease/disorder, wherein the nitroprusside salt or solvate is to be administered in combination with a sulfide salt or a solvate thereof. The invention also provides a sulfide salt or a solvate thereof for use in the treatment or prevention of a cardiovascular disease/disorder, wherein the sulfide salt or solvate is to be administered in combination with a nitroprusside salt or a solvate thereof.

Moreover, the present invention relates to a method of treating or preventing a cardiovascular disease/disorder, the method comprising the administration of a nitroprusside salt or a solvate thereof in combination with a sulfide salt or a solvate thereof, or a pharmaceutical composition comprising a nitroprusside salt or a solvate thereof, a sulfide salt or a solvate thereof and optionally a pharmaceutically acceptable excipient, to a subject in need thereof. The nitroprusside salt or solvate and the sulfide salt or solvate are preferably administered to the subject in therapeutically effective amounts. The subject is preferably a human or a non-human animal, and is more preferably a human.

The nitroprusside salt or solvate and the sulfide salt or solvate may be administered either simultaneously/concomitantly or sequentially (e.g., the nitroprusside salt or solvate may be administered first, followed by the administration of the sulfide salt or solvate, or vice versa). When administration is simultaneous, the nitroprusside salt or solvate and the sulfide salt or solvate may be administered either in the same pharmaceutical composition or in different/separate pharmaceutical compositions. It is preferred that the nitroprusside salt or solvate and the sulfide salt or solvate are provided as separate powders which can be reconstituted for simultaneous administration, e.g., by dissolving both powders in an infusion solution, such as an aqueous solution containing 0.9 wt-% sodium chloride, or lactated Ringer's solution or Hartmann's solution (as described, e.g., in White S.A. et al., 1997), or any other buffered infusion solution. Accordingly, the invention relates to a nitroprusside salt or a solvate thereof in combination with a sulfide salt or a solvate thereof for use in the treatment or prevention of a cardiovascular disease/disorder, wherein the nitroprusside salt or solvate and the sulfide salt or solvate are provided as separate powders.

The nitroprusside salt to be used in accordance with the present invention may be, e.g., an alkali salt of nitroprusside (e.g., sodium nitroprusside or potassium nitroprusside), and is preferably sodium nitroprusside (SNP). Particularly preferred is disodium nitroprusside dihydrate, i.e. Na₂[Fe(CN)₅NO] · 2 H₂O.

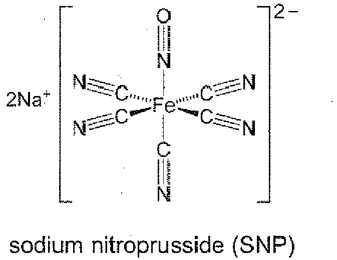

The sulfide salt or solvate is preferably an alkali hydrogensulfide salt (e.g., NaHS or KHS), a dialkali sulfide salt (e.g., Na₂S or K₂S), an alkaline earth metal sulfide salt (e.g., MgS or CaS), or a solvate thereof. More preferably, the sulfide salt or solvate is an alkali hydrogensulfide salt, a dialkali sulfide salt or a solvate thereof, and even more preferably it is sodium hydrogensulfide (NaHS) or sodium sulfide (Na₂S). Most preferably, the sulfide salt is Na₂S. It is to be understood that the term "sulfide salt" as used herein encompasses salts of S²⁻ and salts of HS⁻.

While other compounds capable of releasing/providing H₂S under physiological conditions, such as the H₂S donors known in the art and described in the pertinent literature (e.g., Wallace J.L., 2007; Caliendo G. et al., 2010; Zhao Y. et al., 2011; Hughes M.N. et al., 2009; Kashfi K. et al., 2012), can also be used in combination with a nitroprusside salt or solvate for the treatment or prevention of a cardiovascular disease/disorder, the use of a sulfide salt or solvate in accordance with the present invention is advantageous over the use of such other H₂S donors because the latter release H₂S slowly and are therefore less suitable to render harmless the toxic cyanide that would otherwise be released from nitroprusside.

It is thus particularly preferred that SNP and NaHS are to be used in combination, and it is even more preferred that SNP and Na₂S are to be used in combination.

The sulfide salt or solvate is preferably used in a molar excess as compared to the nitroprusside salt or solvate. It is particularly preferred that the sulfide salt or solvate (such as, e.g., Na₂S or NaHS) is used in an about 2-fold to about 10-fold molar excess, more preferably in an about 3-fold to about 7-fold molar excess, even more preferably in an about 5-fold to about 6-fold molar excess, and yet even more preferably in an about 6-fold molar excess, with respect to the amount of the nitroprusside salt or solvate (e.g., SNP) to be employed.

In the context of the present invention, the reaction of SNP and a sulfide salt has been studied under physiologically relevant conditions using both chemical and physiological/pharmacological tools. It has surprisingly been found that nitroprusside salts such as SNP react directly with H₂S released from sulfide salts to generate nitroxyl (HNO), without any intermediate formation of NO, and to convert the toxic cyanide released from nitroprusside into harmless thiocyanate. As shown in Example 2, the intracellular formation of HNO from SNP and Na₂S has been confirmed and the subsequent release of calcitonin gene-related peptide (CGRP) from mouse heart has been demonstrated. The present invention thus provides a novel HNO-generating therapeutic having potent vasodilatory and positive inotropic effects for the medical intervention of cardiovascular diseases/disorders, which allows to avoid the toxic side effects of SNP resulting from the release of cyanide from nitroprusside and to avoid the emergence of nitrate-tolerance.

The cardiovascular disease/disorder to be treated or prevented in accordance with the present invention is preferably selected from heart failure (such as, e.g., congestive heart failure (e.g., acute congestive heart failure), acute decompensated heart failure, diastolic heart failure, or cardiac asthma), heart attack or myocardial infarction, hypertension (such as, e.g., hypertensive heart disease, hypertensive nephropathy, essential hypertension, secondary hypertension (e.g., renovascular hypertension), pulmonary hypertension, malignant hypertension, benign hypertension, systolic hypertension, white coat hypertension, or acute hypertension), coronary obstruction, coronary artery disease (CAD), angina pectoris, ischemic cardiomyopathy and/or infarction, pulmonary congestion, pulmonary edema, cardiac fibrosis, valvular heart disease, pericardial disease, circulatory congestive state, peripheral edema, ascites, myocarditis, Chagas disease, ventricular hypertrophy, or heart valve disease.

It is particularly preferred that the cardiovascular disease/disorder to be treated or prevented is selected from heart failure (such as, e.g., congestive heart failure (e.g., acute congestive heart failure), acute decompensated heart failure, diastolic heart failure, or cardiac asthma), heart attack, myocardial infarction, or hypertension (such as, e.g., hypertensive heart disease, hypertensive nephropathy, essential hypertension, secondary hypertension (e.g., renovascular hypertension), pulmonary hypertension, malignant hypertension, benign hypertension, systolic hypertension, white coat hypertension, or acute hypertension).

The combination of a nitroprusside salt or a solvate thereof with a sulfide salt or a solvate thereof according to the invention is also useful as a vasodilator, e.g., to promote wound healing or for treating or preventing trauma or physical injury, such as anal fissure. Accordingly, the present invention relates to a nitroprusside salt or a solvate thereof in combination with a sulfide salt or a solvate thereof for use in promoting wound healing and also for use in treating or preventing trauma or physical injury, such as, e.g., anal fissure.

The nitroprusside salt or solvate as well as the sulfide salt or solvate to be used in accordance with the present invention are commercially available and can also be prepared in accordance with or in analogy to synthetic routes described in the literature (e.g., Hyde, F.S., 1897; Brauer, G., 1963).

It is to be understood that any salts to be used as pharmaceuticals or to be contained in a pharmaceutical composition in the present invention, including in particular the nitroprusside salts and the sulfide salts described herein, are preferably pharmaceutically acceptable salts.

Moreover, the invention relates to the nitroprusside salts and the sulfide salts described herein in any solvated form, including, e.g., solvates with water, for example hydrates, or with organic solvents such as, e.g., methanol, ethanol or acetonitrile, i.e., as a methanolate, ethanolate or acetonitrilate, respectively, or in the form of any polymorph.

The nitroprusside salts and the sulfide salts described herein may be administered as compounds *per* se or may be formulated as medicaments. The medicaments/pharmaceutical compositions may optionally comprise one or more pharmaceutically acceptable excipients, such as carriers, diluents, fillers, disintegrants, lubricating agents, binders, colorants, pigments, stabilizers, preservatives, antioxidants, or solubility enhancers.

The pharmaceutical compositions can be formulated by techniques known to the person skilled in the art, such as the techniques published in Remington's Pharmaceutical Sciences, 20th Edition. The pharmaceutical compositions can be formulated as dosage forms for parenteral, such as intramuscular, intravenous, subcutaneous, intradermal, intraarterial, intracardial, rectal, nasal, topical, aerosol or vaginal administration. Dosage forms for parenteral administration include solutions, emulsions, suspensions, dispersions and powders and granules for reconstitution. Emulsions are a preferred dosage form for parenteral administration. Dosage forms for rectal and vaginal administration include suppositories and ovula. Dosage forms for nasal administration can be administered via inhalation and insufflation, for example by a metered inhaler. Dosage forms for topical administration include creams, gels, ointments, salves, patches and transdermal delivery systems. Preferably, the pharmaceutical compositions of the invention are formulated as dosage forms for parenteral administration (such as, e.g., for intravenous, intraarterial, intraperitoneal, intrathecal, intraventricular, intraurethral, intrasternal, intracardial, intracranial or intramuscular administration), particularly for intramuscular or intravenous administration, and most preferably for intravenous infusion.

The nitroprusside salt or solvate and the sulfide salt or solvate according to the invention, or the corresponding pharmaceutical compositions described above, may be administered to a subject by any convenient route of administration, whether systemically/peripherally or at the site of desired action, including but not limited to one or more of: topical (e.g., transdermal, intranasal, ocular, buccal, and sublingual), parenteral (e.g., using injection techniques or infusion techniques, and including, for example, by injection, e.g., subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, or intrasternal by, e.g., implant of a depot, for example, subcutaneously or intramuscularly), pulmonary (e.g., by inhalation or insufflation therapy using, e.g., an aerosol, e.g., through mouth or nose), gastrointestinal, intrauterine, intraocular, subcutaneous, ophthalmic (including intravitreal or intracameral), rectal, and vaginal. The parenteral route of administration is particularly envisaged, with intramuscular or intravenous administration being preferred, and intravenous infusion being most preferred.

Accordingly, the nitroprusside salt or solvate and the sulfide salt or solvate according to the invention, or corresponding pharmaceutical compositions, are preferably administered parenterally. Examples of such administration include one or more of: intravenously, intraarterially, intraperitoneally, intrathecally, intraventricularly, intraurethrally, intrasternally, intracardially, intracranially or intramuscularly administering the compounds or pharmaceutical compositions, and/or by using infusion techniques. For parenteral administration, the compounds are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

The nitroprusside salt or solvate and the sulfide salt or solvate according to the invention, or corresponding pharmaceutical compositions, may also be administered by the pulmonary route, rectal routes, or the ocular route. For ophthalmic use, they can be formulated as micronized suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For topical application to the skin, the nitroprusside salt or solvate and the sulfide salt or solvate according to the invention, or corresponding pharmaceutical compositions, can be formulated as a suitable ointment containing the active compound(s) suspended or dissolved in, for example, a mixture with one or more of the following: mineral oil, liquid petrolatum, white petrolatum, propylene glycol, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, 2-octyldodecanol, benzyl alcohol and water.

It is particularly preferred that the combination of a nitroprusside salt or a solvate thereof with a sulfide salt or a solvate thereof according to the present invention is to be administered parenterally, in particular by intravenous injection or infusion, and most preferably by intravenous infusion.

Typically, a physician will determine the actual dosage which will be most suitable for an individual subject. The specific dose level and frequency of dosage for any particular individual subject may be varied and will depend upon a variety of factors including the activity of the specific compounds/salts employed, the metabolic stability and length of action of these compounds/salts, the age, body weight, general health, sex, diet, mode and time of administration, rate of excretion, drug combination, the severity of the particular condition, and the individual subject undergoing therapy.

A proposed, yet non-limiting dose of the combination of a nitroprusside salt or solvate with a sulfide salt or solvate according to the invention for parenteral (particularly intravenous) administration to an animal (e.g., a human) is in the range of about 1 µg to about 1 mg of the nitroprusside salt or solvate (e.g., SNP) per kg body weight per day in combination with about 1 µg to about 5 mg of the sulfide salt or solvate (e.g., NaHS or Na₂S) per kg body weight per day, including in particular a dose in the range of about 10 µg/kg/day to about 1 mg/kg/day of the nitroprusside salt or solvate in combination with about 5 µg/kg/day to about 7 mg/kg/day of the sulfide salt or solvate. The combination of the nitroprusside salt or solvate with the sulfide salt or solvate may be administered, e.g., by continuous intravenous infusion or by 1 to 4 injections per day. If administered continuously, the combination according to the invention may be administered at a dose rate of about 0.5 µg/kg/min to about 100 µg/kg/min. It will be appreciated that it may be necessary to make routine variations to the dosage depending on the age and weight of the patient/subject as well as the severity of the condition to be treated. The precise dose and also the route of administration will ultimately be at the discretion of the attendant physician or veterinarian.

The combination of a nitroprusside salt or a solvate thereof with a sulfide salt or a solvate thereof according to the present invention may be administered in the context of a monotherapy (i.e., using the nitroprusside salt or solvate and the sulfide salt or solvate as the sole pharmaceutically active agents for the treatment or prevention of a cardiovascular disease/disorder) or in combination with one or more further pharmaceutically active agents. When the nitroprusside salt or solvate and the sulfide salt or solvate according to the invention are used in combination with a further pharmaceutically active agent which is active against the same cardiovascular disease/disorder, a lower dose of each agent may be used. The combination of the nitroprusside salt or solvate and the sulfide salt or solvate according to the present invention with one or more further pharmaceutically active agents may comprise the simultaneous/concomitant administration of the further pharmaceutically active agents with the nitroprusside salt or solvate and the sulfide salt or solvate according to the invention. However, sequential/separate administration is also envisaged.

Preferably, the further pharmaceutically active agent to be administered in combination with the nitroprusside salt or solvate and the sulfide salt or solvate according to the present invention is an agent for the treatment or prevention of a cardiovascular disease or disorder, such as, e.g., a vasodilator, a positive inotropic agent, a diuretic, or a beta-adrenergic receptor antagonist.

The vasodilator may, e.g., be selected from glyceryl trinitrate, isosorbide dinitrate, isosorbide mononitrate, linsidomine, molsidomine, pentaerythritol tetranitrate, propatylnitrate, tenitramine, trolnitrate, flosequinan, itramin tosilate, prenylamine, oxyfedrine, benziodarone, carbocromen, hexobendine, etafenone, heptaminol, imolamine, dilazep, trapidil, molsidomine, efloxate, cinepazet, cloridarol, nicorandil, nesiritide, gapicomine, pimobendan, or levosimendan.

The positive inotropic agent may, e.g., be selected from berberine, levosimendan, omecamtiv, dopamine, dobutamine, dopexamine, epinephrine (adrenaline), isoprenaline (isoproterenol), norepinephrine (noradrenaline), digoxin, digitalis, prostaglandins, phosphodiesterase inhibitors (such as, e.g., enoximone, milrinone, amrinone, or theophylline), glucagon, or insulin.

The diuretic may, e.g., be selected from acetazolamide, furosemide, bumetanide, etacrynic acid, etozoline, muzolimine, piretanide, tienilic acid, torasemide, hydrochlorothiazide, bendroflumethiazide, hydroflumethiazide, chlorothiazide, polythiazide, trichlormethiazide, cyclopenthiazide, methyclothiazide, cyclothiazide, mebutizide, quinethazone, clopamide, chlortalidone, mefruside, clofenamide, metolazone, meticrane, xipamide, indapamide, clorexolone, fenquizone, amiloride, triamterene, benzamil, spironolactone, eplerenone, potassium canrenoate, canrenone, mannitol, urea, conivaptan, mozavaptan, satavaptan, tolvaptan, demeclocycline, mersalyl, meralluride, theobromine, or cicletanine.

The beta-adrenergic receptor antagonist ("beta blocker") may, e.g., be selected from alprenolol, bopindolol, bupranolol, carteolol, cloranolol, mepindolol, nadolol, oxprenolol, penbutolol, pindolol, propranolol, sotalol, tertatolol, timolol, acebutolol, atenolol, betaxolol, bevantolol, bisoprolol, celiprolol, epanolol, esmolol, nebivolol, metoprolol, practolol, S-atenolol, talinolol, carvedilol, labetalol, or butaxamine.

The combinations referred to above may conveniently be presented for use in the form of a pharmaceutical formulation. The individual components of such combinations may be administered either sequentially or simultaneously/concomitantly in separate or combined pharmaceutical formulations by any convenient route. When administration is sequential, either the combination of a nitroprusside salt or a solvate thereof with a sulfide salt or a solvate thereof according to the invention or the further pharmaceutically active agent may be administered first. When administration is simultaneous, the combination including the further pharmaceutically active agent may be administered either in the same or different pharmaceutical compositions. When combined in the same formulation, it will be appreciated that the various compounds must be stable and compatible with each other and the other components of the formulation. When formulated separately, they may be provided in any convenient formulation, conveniently in such manner as is known for such compounds in the art.

Accordingly, the present invention relates to a nitroprusside salt or a solvate thereof for use in the treatment or prevention of a cardiovascular disease/disorder, wherein the nitroprusside salt or solvate is to be administered in combination with a sulfide salt or a solvate thereof and with a further pharmaceutically active agent (such as, e.g., a vasodilator, a positive inotropic agent, a diuretic, or a beta-adrenergic receptor antagonist). The invention also provides a sulfide salt or a solvate thereof for use in the treatment or prevention of a cardiovascular disease/disorder, wherein the sulfide salt or solvate is to be administered in combination with a nitroprusside salt or a solvate thereof and with a further pharmaceutically active agent (such as, e.g., a vasodilator, a positive inotropic agent, a diuretic, or a beta-adrenergic receptor antagonist). The invention furthermore relates to a pharmaceutical composition for use in the treatment or prevention of a cardiovascular disease/disorder, the pharmaceutical composition comprising a nitroprusside salt or a solvate thereof in combination with a sulfide salt or a solvate thereof and optionally a pharmaceutically acceptable excipient, wherein the pharmaceutical composition is to be administered in combination with a further pharmaceutically active agent (such as, e.g., a vasodilator, a positive inotropic agent, a diuretic, or a beta-adrenergic receptor antagonist).

The subject or patient, such as the subject in need of treatment or prevention, may be an animal (e.g., a non-human animal), a vertebrate animal, a mammal, a rodent (e.g., a guinea pig, a hamster, a rat, a mouse), a murine (e.g., a mouse), a canine (e.g., a dog), a feline (e.g., a cat), an equine (e.g., a horse), a primate, a simian (e.g., a monkey or ape), a monkey (e.g., a marmoset, a baboon), an ape (e.g., a gorilla, chimpanzee, orang-utan, gibbon), or a human. The meaning of the terms "animal", "mammal", etc. is well known in the art and can, for example, be deduced from Wehner und Gehring (1995; Thieme Verlag). In the context of this invention, it is also envisaged that animals are to be treated which are economically, agronomically or scientifically important. Scientifically important organisms include, but are not limited to, mice, rats, and rabbits. Non-limiting examples of agronomically important animals are sheep, cattle and pigs, while, for example, cats and dogs may be considered as economically important animals. Preferably, the subject/patient is a mammal. More preferably, the subject/patient is a human or a non-human mammal (such as, e.g., a guinea pig, a hamster, a rat, a mouse, a rabbit, a dog, a cat, a horse, a monkey, an ape, a marmoset, a baboon, a gorilla, a chimpanzee, an orang-utan, a gibbon, a sheep, cattle, or a pig). Even more preferably, the subject/patient is a human.

The term "treatment of a disorder or disease" as used herein is well known in the art. "Treatment of a disorder or disease" implies that a disorder or disease is suspected or has been diagnosed in a patient/subject. A patient/subject suspected of suffering from a disorder or disease typically shows specific clinical and/or pathological symptoms which a skilled person can easily attribute to a specific pathological condition (i.e., diagnose a disorder or disease).

"Treatment of a disorder or disease" may, for example, lead to a halt in the progression of the disorder or disease (e.g., no deterioration of symptoms) or a delay in the progression of the disorder or disease (in case the halt in progression is of a transient nature only). "Treatment of a disorder or disease" may also lead to a partial response (e.g., amelioration of symptoms) or complete response (e.g., disappearance of symptoms) of the subject/patient suffering from the disorder or disease. "Amelioration" of a disorder or disease may, for example, lead to a halt in the progression of the disorder or disease or a delay in the progression of the disorder or disease. Such a partial or complete response may be followed by a relapse. It is to be understood that a subject/patient may experience a broad range of responses to a treatment (e.g., the exemplary responses as described herein above).

Treatment of a disorder or disease may, inter alia, comprise curative treatment (preferably leading to a complete response and eventually to healing of the disorder or disease) and palliative treatment (including symptomatic relief).

Also the term "prevention of a disorder or disease" as used herein is well known in the art. For example, a patient/subject suspected of being prone to suffer from a disorder or disease as defined herein may, in particular, benefit from a prevention of the disorder or disease. The subject/patient may have a susceptibility or predisposition for a disorder or disease, including but not limited to hereditary predisposition. Such a predisposition can be determined by standard assays, using, for example, genetic markers or phenotypic indicators. It is to be understood that a disorder or disease to be prevented in accordance with the present invention has not been diagnosed or cannot be diagnosed in the patient/subject (for example, the patient/subject does not show any clinical or pathological symptoms). Thus, the term "prevention" comprises the use of compounds of the present invention before any clinical and/or pathological symptoms are diagnosed or determined or can be diagnosed or determined by the attending physician.

The term "about" refers to ±10% of the indicated numerical value, and in particular to ±5% of the indicated numerical value. Whenever the term "about" is used, a specific reference to the exact numerical value indicated is also included. For example, the expression "about 100" refers to the range of 90 to 110, in particular the range of 95 to 105, and preferably refers to the specific value of 100.

In this specification, a number of documents including patent applications and scientific literature are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The invention is also illustrated by the following illustrative figures. The appended figures show:

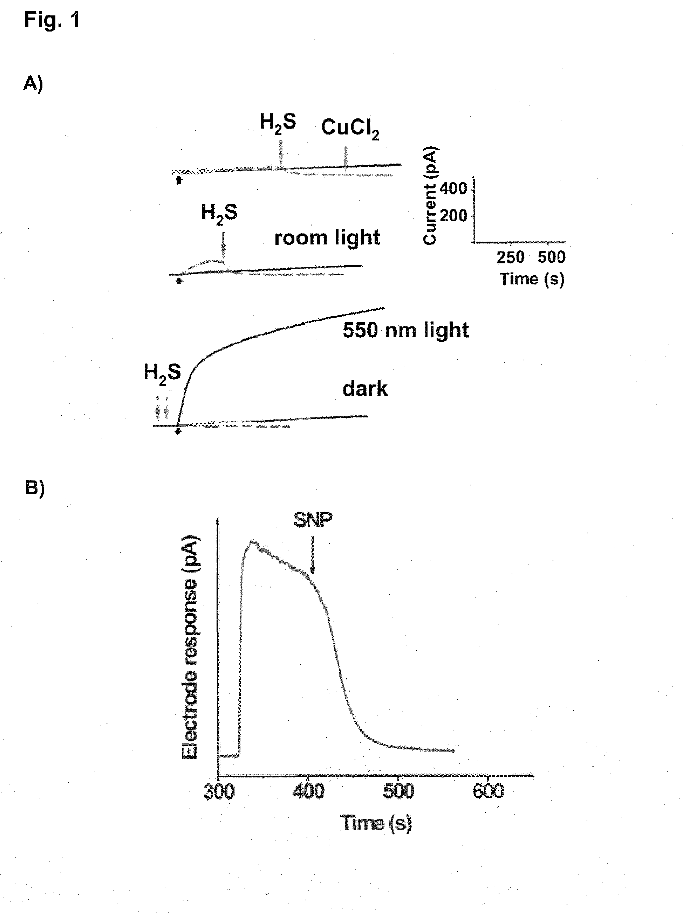

**Figure 1****:** SNP efficiently removes H₂S from the solution without any NO release. (**A**) The NO electrode trace of the reaction between H₂S and SNP. The upper panel represents the response of the NO electrode (in the dark) to the addition of 100 µM SNP in a buffered solution (straight line), and the response upon the addition of 100 µM of both Na₂S and CuCl₂ (dashed line). The middle panel shows the effect of H₂S on the ambient light-induced release of NO from SNP (dashed line). The electrode response to SNP in the absence of light is shown for comparison (straight line). The lower panel depicts the effect of H₂S on the light-induced NO release from SNP. The addition of Na₂S to a buffered solution exposed to a 500-550 nm light source (dashed line) or in the absence of light (dashed line) prior to the addition of SNP completely abolished the NO release. The straight lines represent the corresponding responses upon the addition of only SNP. The points in time at which H₂S or CuCl₂ were added are marked with arrows. The small arrow at the bottom of each panel denotes the point in time at which SNP was added. **(B)** H₂S electrode traces for the reaction of H₂S with SNP. A 50 µM Na₂S solution was injected into a 300 mM phosphate buffer at a pH 7.4 under aerobic conditions followed by the injection of an equimolar amount of SNP.

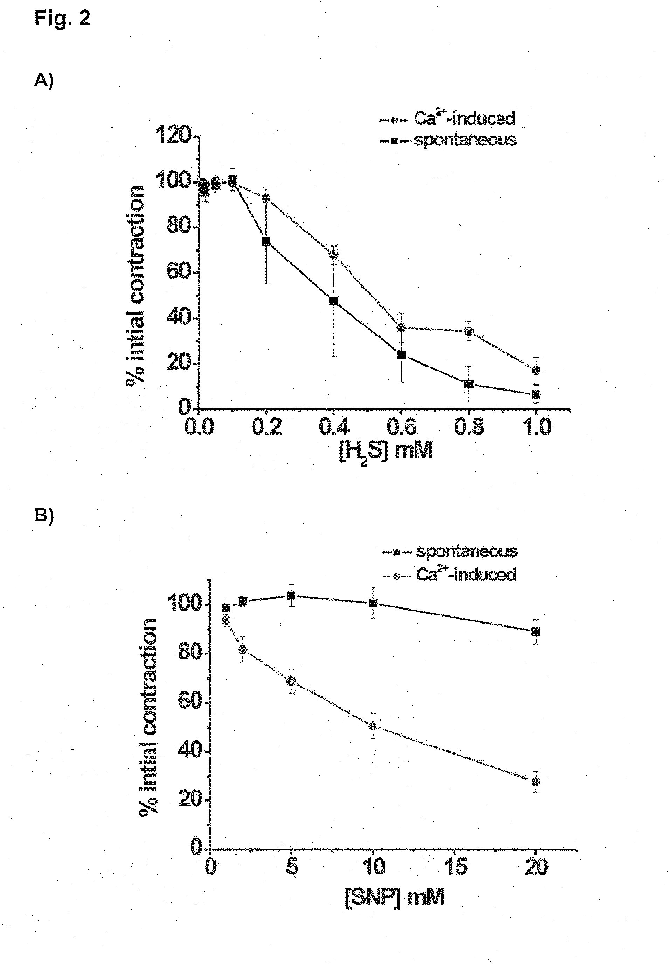

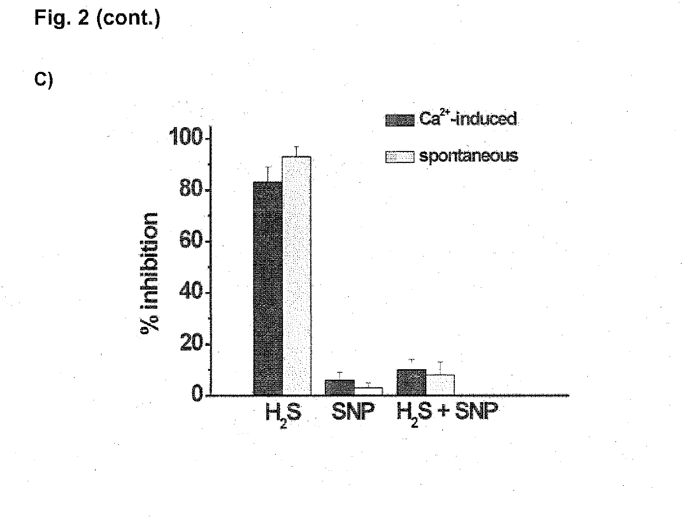

**Figure 2****:** Spontaneous and Ca²⁺ induced contractile activity of rat uteri treated with H₂S **(A)** or SNP **(B).** The contractile activity was expressed as the relative ratio between the mean height peak of the untreated control and the treated uteri. The data are expressed as the mean ± SEM (n = 8). **(C)** The effects of 1 mM H₂S, 1 mM SNP and the combination of both on the spontaneous and Ca²⁺ induced uterus contractions.

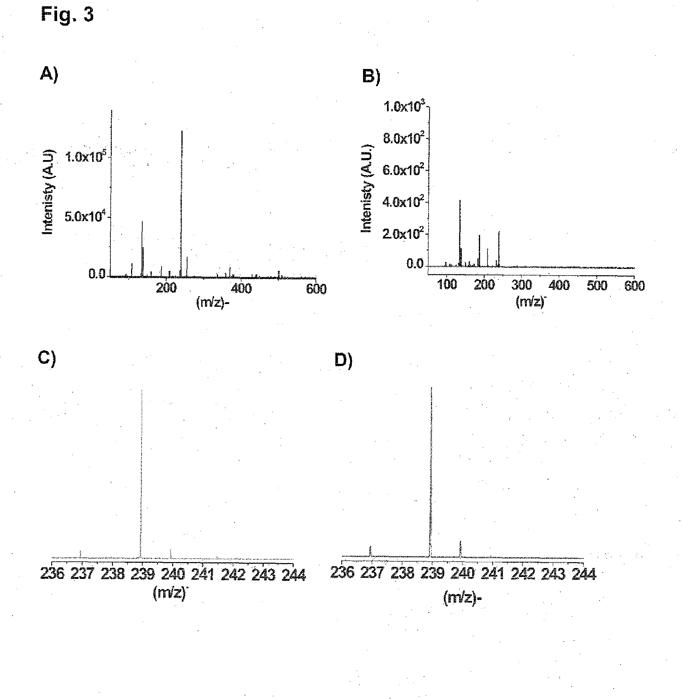

**Figure 3****:** Ultra-high resolution cryo-spray (4 °C) ESI-TOF-mass spectrometry in negative mode of SNP **(A)** and of the reaction mixture containing 1mM SNP and 5 mM H₂S in 50 mM KPi pH 7.4 **(B).** The reaction mixture shows only small traces of starting compound (the peak intensity is more than 250 times lower than that of 1 mM SNP). Isotopic distribution of the observed peak **(C)** at m/z 238.9 assigned to Na[Fe(CN)₅NO]⁻ and simulation of isotopic distribution for the same molecule **(D).**

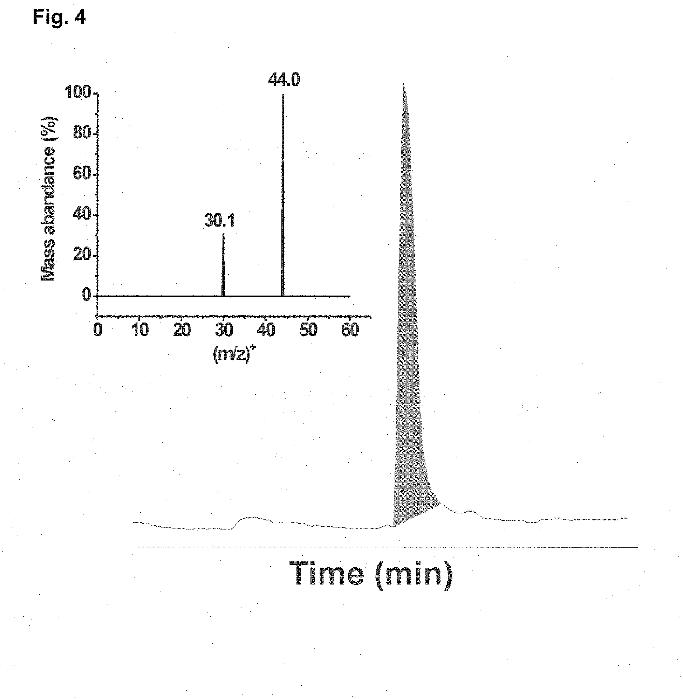

**Figure 4****:** GC-MS detection of N₂O generation from SNP and H₂S reaction mixture (1:5). 2 mM SNP in 50 mM KPi pH 7.4 was degassed with argon and kept in dark glass vials sealed with PTFE septa. Na₂S was added to yield a final concentration of 10 mM. GC-MS analyses were performed on a Bruker GC 450 TQ MS 300. The gas chromatograph was equipped with capillary column Varian, VF-5m. 50 µL of headspace gas samples were injected in the splitless mode. The following oven temperature program was used with helium as the carrier gas: ramp from 50 to 155 °C at a rate of 10 °C/min and then to 260 °C at a rate of 30 °C/min. Positive electron impact ionization mode was used. Detector multiplier voltage was set to 1400 V and the detection performed by selected ion monitoring of *m*/*z* 44 (N₂O) and *m*/*z* 30 (NO) using a dwell time of 50 ms and scan width for SIM of 0.7 a.u. Areas under the peaks were determined using the software provided by the manufacturer. The inset represents the characteristic MS spectrum of the observed peak.

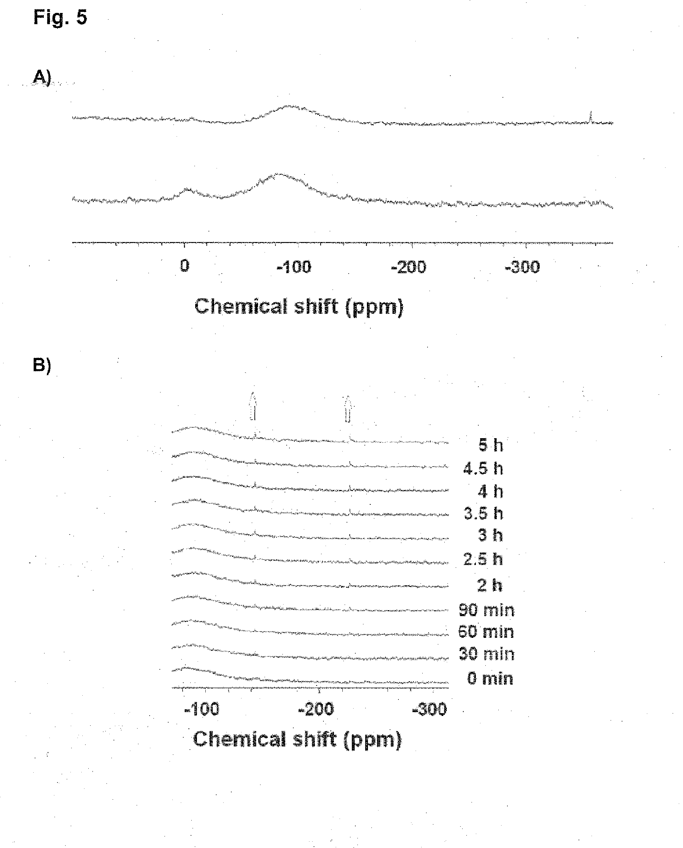

**Figure 5****:** ¹⁴NMR spectroscopic analysis of the reaction between 200 mM Na₂S and 100 mM SNP at pH 7.4. (**A**) The entire range of the ¹²N NMR spectra for 100 mM SNP in 300 mM KPi at pH 7.4 (lower line), and the spectrum of the reaction mixture containing 100 mM of both SNP and Na₂S in 300 mM KPi at pH 7.4 (upper line). (**B**) The time dependent detection of N₂O formation based on its characteristic chemical shifts at -142 ppm and -225 ppm.

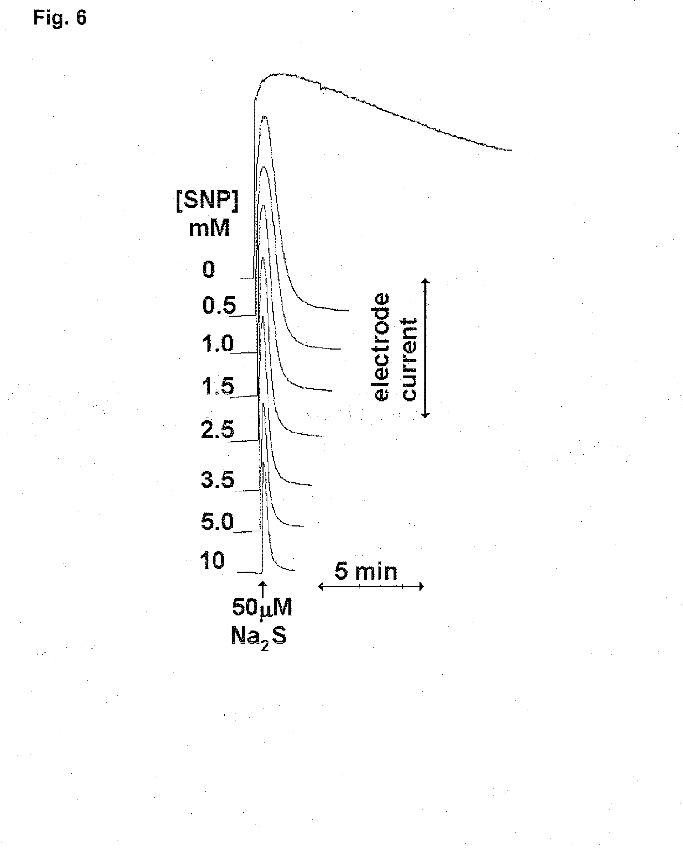

**Figure 6****:** Representative traces of H₂S electrode responses upon addition of 50 µM Na₂S into buffered solution containing increasing concentrations of SNP (0-10 mM).

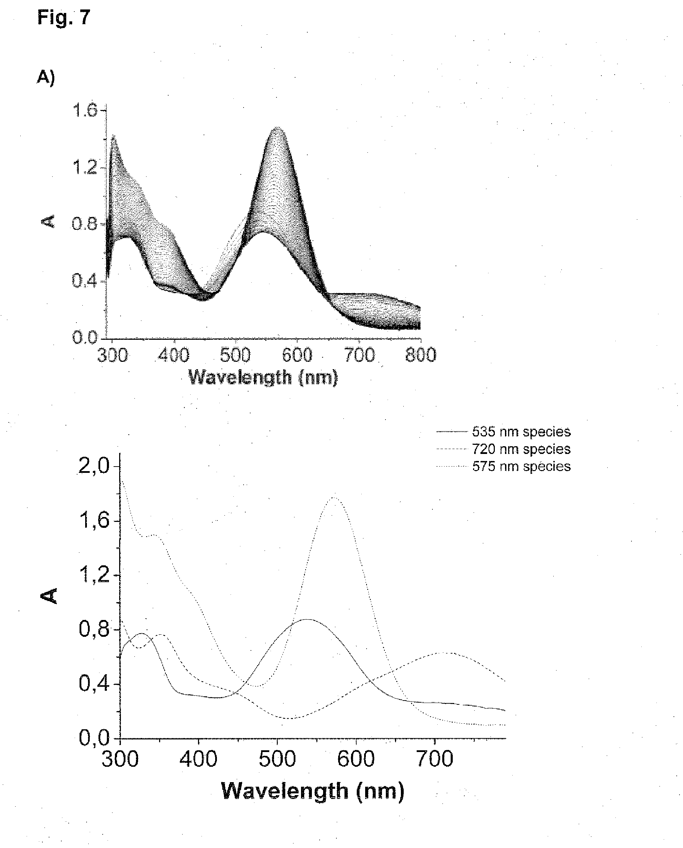

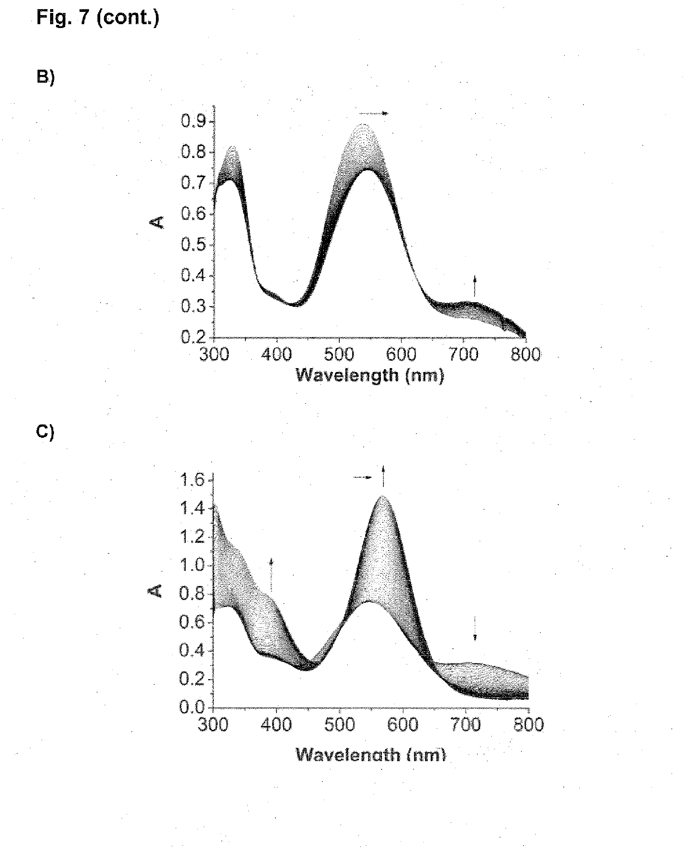

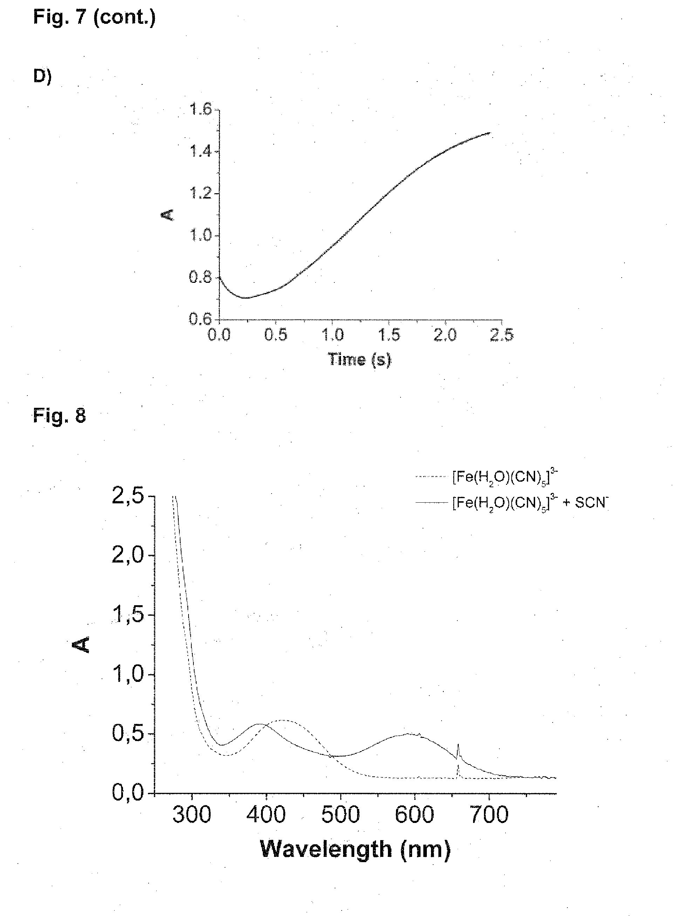

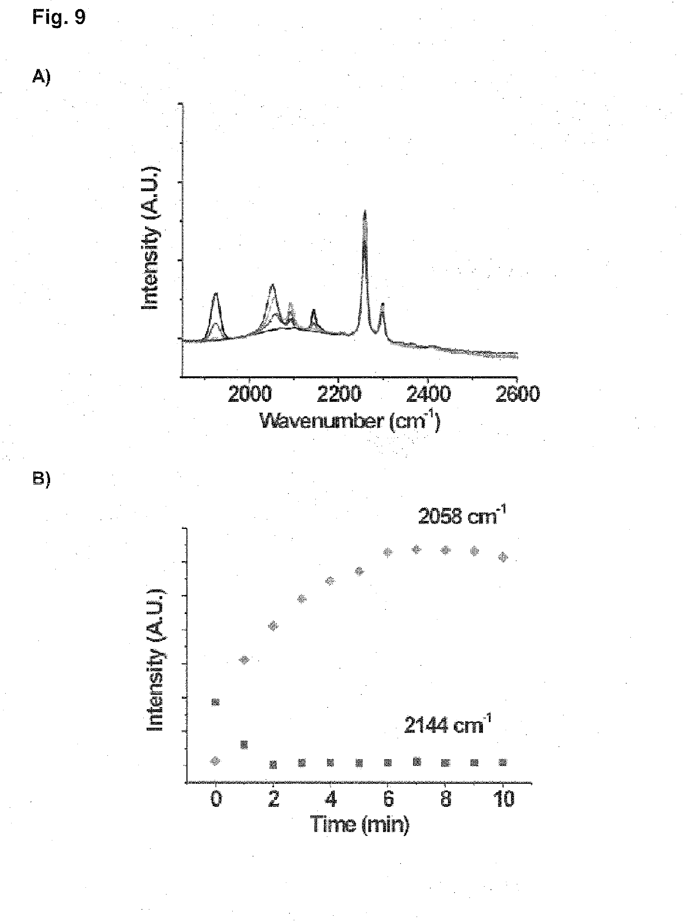

**Figure 7****:** Kinetic analysis of the reaction between SNP (200 µM) and Na₂S (25 mM) in 300 mM KPi pH 7.4. (**A**) The monitoring of this reaction (under aerobic conditions) using time resolved UV/Vis spectroscopy coupled to a stopped-flow device (total observation time 2.6 s, integration time 2.6 ms); at the bottom of Figure 7A, spectra of the intermediates (535 nm species (straight line) = [(CN)₅FeN(O)SH]³⁻, 720 nm species (dashed line) = mixture of a Prussian blue type compounds, transient [(CN)₅Fe(HNO)]³⁻) and the final product [Fe^{II}(CN)₅₋ₓ(SCN)ₓ(H₂O)]³⁻; 575 nm species (dotted line) = final product [Fe^{II}(CN)₅₋ₓ((SCN)ₓ(H₂O)]³⁻) over the course of the reaction based on the global spectra analysis (SpecFit) are shown. (**B**) The second reaction step (the entire reaction is presented in Figure 9A), which shows constant decrease and shifting of the 535 nm peak and the appearance of an absorbance at ~720 nm. The first reaction step, i.e., the formation of the 535 nm species, was too fast to be detected. (**C**) The third reaction step during which a further shift and buildup of the 575 nm species is observed with a decrease in the absorbance maximum at 720 nm. (**D**) A kinetic trace of the peak at 575 nm clearly shows the induction period for the catalytic reactions.
**Figure 8****:** The spectrum of the (Fe(H₂O)(CN)₅]³⁻ before (dashed line) and after (straight line) the addition of an excess of potassium thiocyanate.

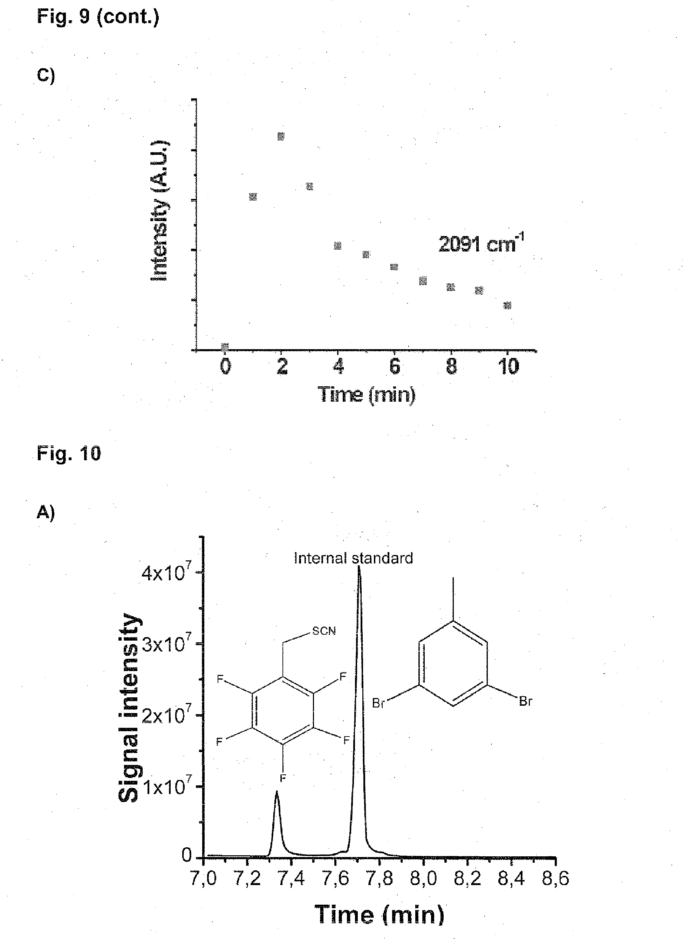

**Figure 9****:** Real-time IR spectroscopic study of the reactions between 150 mM Na₂S and 100 mM SNP at pH 7.4. (**A**) IR spectral changes observed upon the addition of Na₂S into a buffered SNP solution. (**B-C**) The time dependence of the characteristic stretching vibrations (2058 and 2144 cm⁻¹ on **B**, and 2091 cm⁻¹ on **C**) upon the addition of an equimolar concentration of Na₂S into a buffered 100 mM SNP solution as measured by reaction IR spectroscopy.

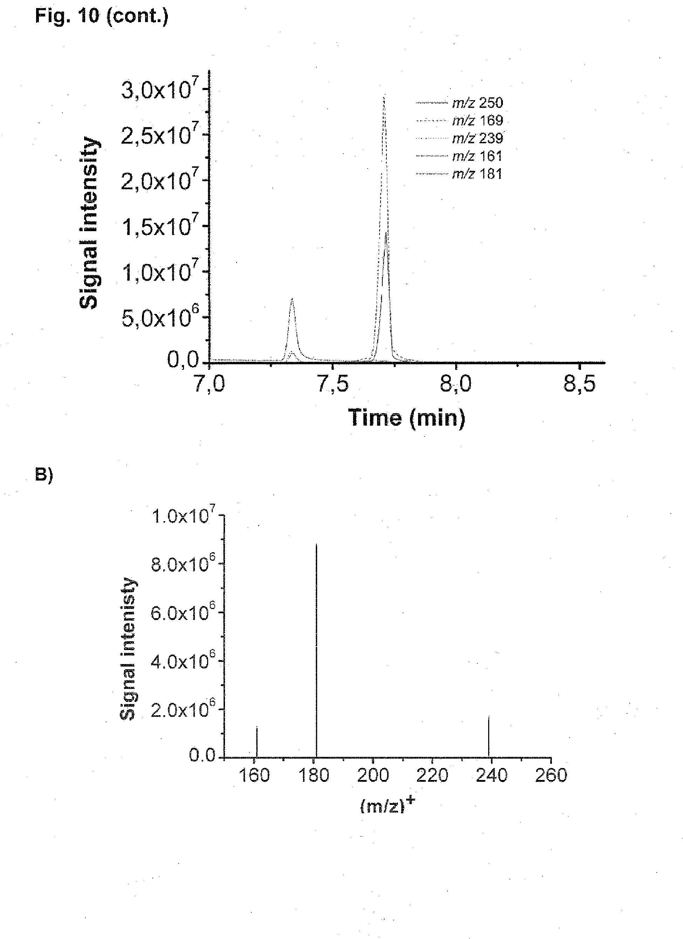

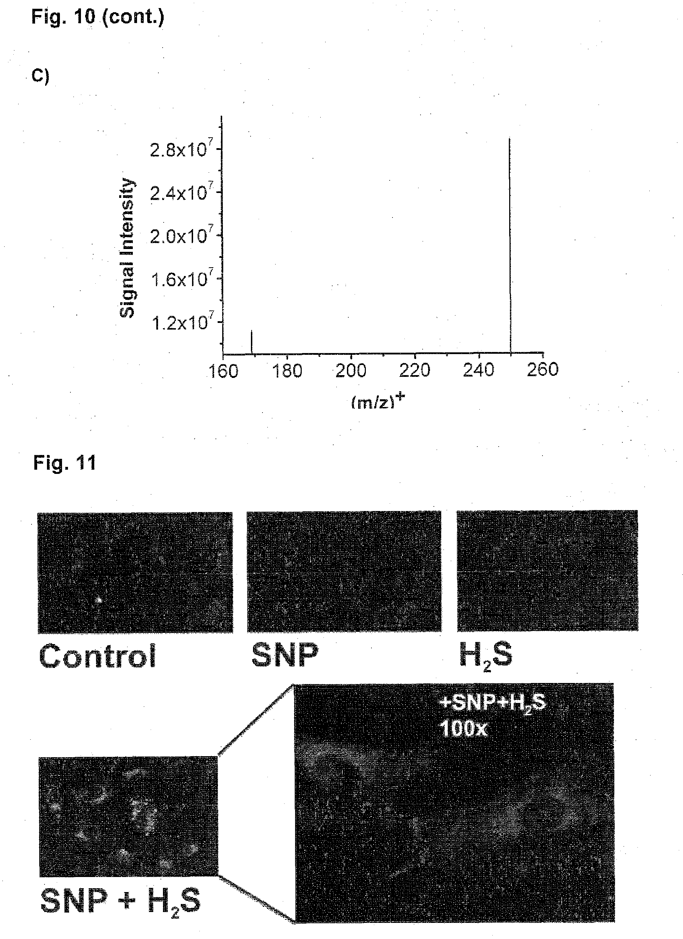

**Figure 10****:** GC-MS analysis of the thiocyanate coupled to pentafluorobenzyl bromide in extractive alkylation process. 1 mM SNP was mixed with 2 mM Na₂S in phosphate buffer pH 7.4 and after 5 min subjected to extractive alkylation as described in Example 1. (**A**) Total GC-MS obtained for selected ion mode detection (*m*/*z* 239, 181, 161 for the pentafluorobenzyl thiocyanate product, and *m*/*z* 250, 169 for the internal standard, 3,5-dibromotoluene). The individual chromatograms of these ions are shown at the bottom of Fig. 10A, with 239, 181, 161 appearing at 7.3 min and 250 and 169 appearing at 7.7 min. (**B**) MS spectrum of the peak at 7.3 min. (**C**) MS spectrum of the peak at 7.7 min.
**Figure 11****:** Intracellular fluorescence in HUVECs, caused by HNO formation. After loading with the HNO sensor, CuBOT1, the cells were treated with either 100 µM Na₂S, 100 µM SNP, or a combination of both. The photomicrographs were taken at a x40 magnification. The representative photomicrograph for the cells treated with a combination of Na₂S and SNP is also shown at a x100 magnification.

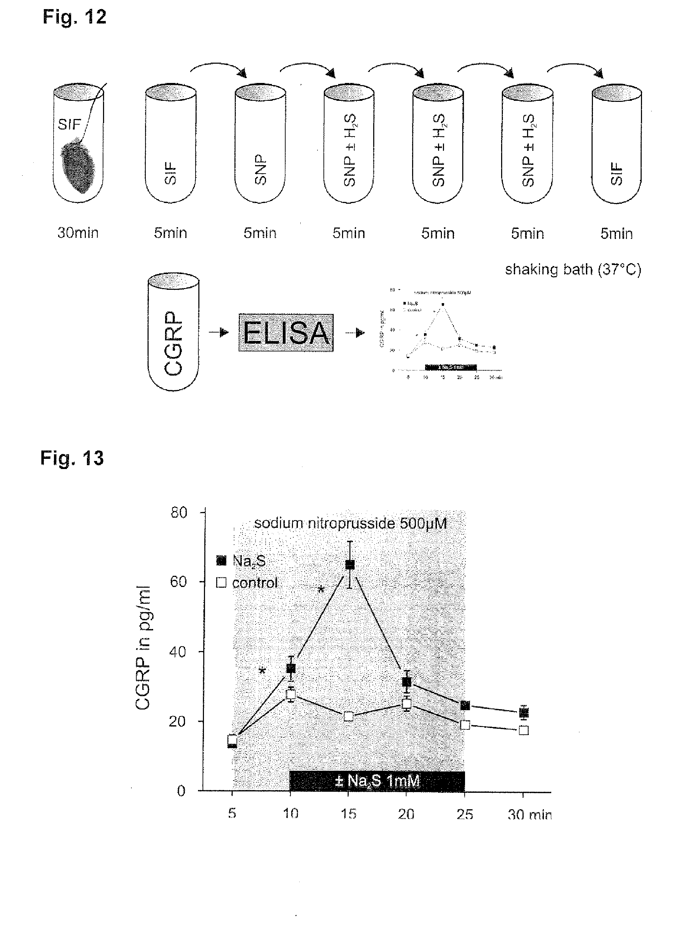

**Figure 12****:** Schematic representation of the experimental setup for detecting CGRP release from isolated heart.
**Figure 13****:** HNO-induced release of CGRP from an isolated mouse heart upon stimulation with a reaction mixture containing 500 µM SNP and 1 mM Na₂S.

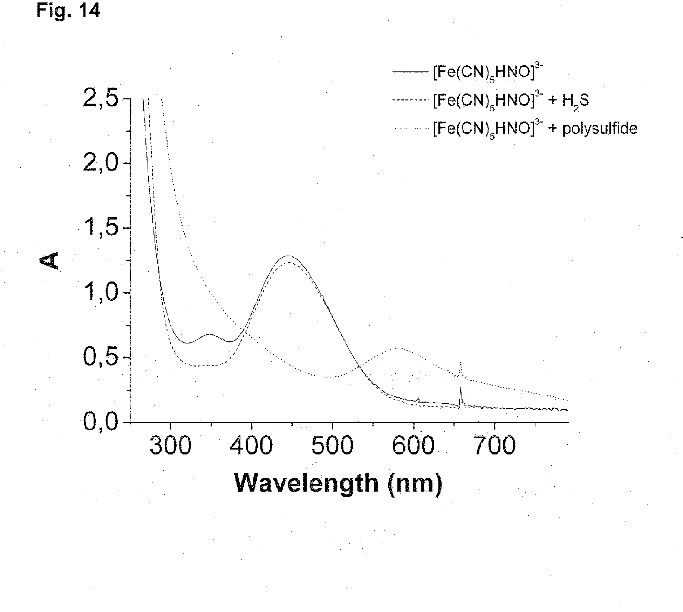

**Figure 14****:** The spectrum of [Fe(CN)₅HNO]³⁻ (straight line) generated by total reduction of SNP with two equivalents of dithionite at pH 7.4 (Montenegro, A.C. et al., 2009). Addition of the excess of sodium sulfide (dashed line) does not change the spectral properties, while the addition of sodium polysulfide gives immediate blue coloration with absorption maximum at ~570 nm (dotted line).

The invention will now be described by reference to the following examples which are merely illustrative and are not to be construed as a limitation of the scope of the present invention.

### EXAMPLES

Unless stated otherwise, all chemicals were purchased from Sigma Aldrich. Na₂S was purchased in the anhydrous form and was both opened and stored in a glove box (<1 ppm O₂ and <1 ppm H₂O). In all experiments described in the following, Na₂S was used to provide H₂S. The solutions were prepared and handled as in described in Filipovic, M.R. et al., 2012, c.

### Example 1: Reaction of SNP with H₂S

### 1) General notes on the reaction of SNP with thiols

As illustrated in Scheme 1 below, the general mechanism for the reactions of SNP with thiols involves the initial formation of a coordinated S-nitrosothiol, which has the characteristic pink color that has long been used as a proof for thiols in Brand's assay (Brand, E. et al., 1930). The S-nitrosothiols (RSNOs) formed account for the "NO-like" physiology of SNP because they can be either homolytically cleaved to give NO (Grossi, L. et al., 2005; Szacilowski, K. et al., 2002), corresponding to pathway a in Scheme 1, or they can be released to further react with the corresponding physiological targets (Butler, A. R. et al., 2002; Roncaroli, F. et al., 2007; Butler, A. R. et al., 1988; Grossi, L. et al., 2005; Johnson, M. D. et al., 1983), as reflected by pathway b in Scheme 1. 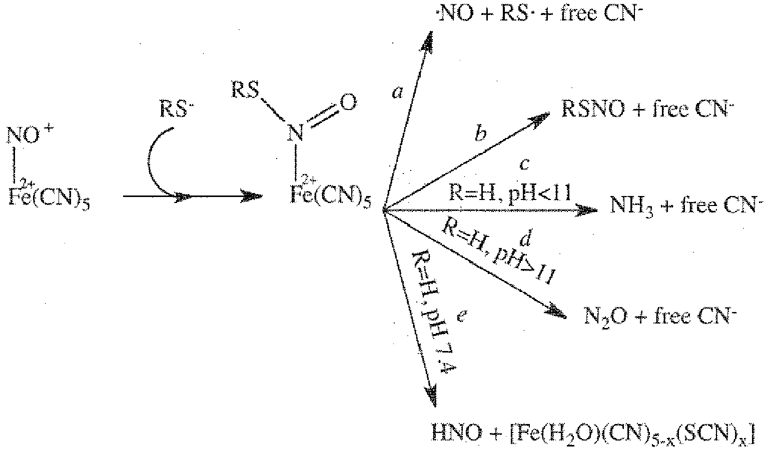
- **Scheme 1**:: Proposed products for the reaction between SNP and different thiols or H₂S. Low molecular weight thiols (cysteine and glutathione) are thought to react with SNP to yield either NO (Szacilowski, K. et al., 2002) (**pathway a**) and/or the corresponding S-nitrosothiols (Grossi, L. et al., 2005) (**pathway *b***). The reaction with H₂S is thought to yield NH₃ if the pH of the solution is less than 11 (**pathway *c***) or N₂O when the pH value is above 11 (**pathway *d***) (Quiroga, S.L. et al., 2011). Throughout all of these studies, the release of free cyanide was proposed. In the context of the present invention, however, the generation of HNO and thiocyanate was demonstrated at a physiological pH and under aerobic conditions (**pathway *e***; x = 1-5), as will be discussed further below.

A chemical study of the reactions of H₂S with SNP has recently appeared (Quiroga, S.L. et al., 2011) and describes the reaction mechanism as initially being similar to that of thiols (although at pH 9-11) with the resulting pink-colored product characteristic of the HSNO/SNO⁻ coordinated adduct of SNP. The end products of this reaction were found to be NH₃ (at pH 8.5-11) and N₂O (at pH > 11), corresponding to pathways c and d in Scheme 1. However, the characterization of some of the intermediates reported in the literature was subsequently questioned (Filipovic, M. R. et al., 2012, a). Notably, both the *in vitro* and *in vivo* generation of and reactions with free (non-coordinated) HSNO/SNO⁻, the smallest S-nitrosothiol, have been recently reported (Filipovic, M.R. et al., 2012, b; Kemsley, J., 2012).

However, it has been shown in the context of the present invention that, under physiologically relevant conditions (aerobic conditions at pH = 7.4), the H₂S and SNP reaction mixture forms a blue product solution. This result is obviously different from the pink/purple products reported for reactions with cysteine, glutathione or H₂S (under anaerobic and alkaline conditions), which implies that a different reaction mechanism occurred.

### 2) Analysis of the reaction of SNP with H₂S by amperometric detection of NO and H₂S

In the literature dealing with the physiology and pharmacology of H₂S, there is a prevailing opinion (Ali, M.Y. et al., 2006; Whiteman, M. et al., 2006) that H₂S and NO react to form a stable S-nitrosothiol that decomposes upon the addition of copper ions to release NO.

To both test the hypothesis of stable *S*-nitrosothiol formation and determine the actual source of the NO release, the reaction between SNP and H₂S was followed using an NO-sensitive electrode both in the total absence of light and under ambient light. The fate of NO was monitored throughout the course of the reaction using ISO-NO probes connected to a Free Radical Analyzer (World Precision Instruments) (Filipovic, M.R. et al., 2012, b).

As expected, there was no release of NO from SNP in the dark, and the addition of H₂S had no effect. Minor amounts of NO were released over time when SNP was exposed to ambient light; however, H₂S addition completely suppressed further NO release, as also shown in Figure 1A. Because it is known that light induces homolytic S-N bond breakage in S-nitrosothiols (Stamler, J.S. et al., 1996), an equimolar mixture of SNP (1 mM) and H₂S (1 mM) was additionally irradiated under a visible light source (500-550 nm). However, no NO release was observed relative to the control, which consisted of only 1 mM SNP and released substantial amounts of NO (Figure 1A). Furthermore, the addition of copper ions into the reaction mixture did not stimulate NO release either (Figure 1A). If the product were an S-nitrosothiol, the immediate release of NO would be expected upon both the addition of copper ions and irradiation. These results strongly suggest that the reaction between SNP and H₂S produces neither a free S-nitrosothiol nor NO.

Next, the effect of SNP on H₂S removal from the solution was followed using a 2 mm shielded H₂S-sensitive electrode (Filipovic, M.R. et al., 2012, b). As shown in Figure 1B, the equimolar addition of SNP completely removed H₂S, which implies that H₂S was consumed rather quickly.

### 3) Organ bath studies on isolated rat uteri to detect potential NO release from SNP during the reaction with H₂S

The effects of H₂S, SNP and their combination on the spontaneous and Ca²⁺-induced contraction of isolated rat uterus preparations were tested. This particular pharmacological model was chosen because, unlike other models, it is relatively resistant (at high millimolar concentration) to SNP-induced dilatation (Hennan, J. K. et al., 1998) but still sensitive to other, real NO donors (Demirkoprulu, N. et al., 2005), which makes it suitable for testing the potential NO release from the SNP and H₂S reaction mixture.

Isolated uteri from virgin Wistar rats (200-250 g) in the oestrus phase of the estrous cycle, as determined by examining a daily vaginal lavage, were used (Appiah, I. et al., 2010). All of the rats were sacrificed by cervical dislocation following a procedure approved by the animal protection authorities (University of Belgrade, Serbia). The uterine horns were rapidly excised and carefully cleaned of any surrounding connective tissue and mounted vertically in a 10 mL volume organ bath containing De Jalon's solution (in g·L⁻¹: NaCl 9.0, KCl 0.42, NaHCO₃ 0.5, CaCl₂ 0.06 and glucose 0.5) aerated with 95% oxygen and 5% carbon dioxide at 37°C. The uteri, either acting spontaneously or being contracted with Ca²⁺ (6 mM), were allowed to equilibrate at 1 g tension before adding the test drugs. Both H₂S and SNP were added cumulatively. The myometrial tension was recorded isometrically using a TSZ-04-E isolated organ bath and transducer (Experimetria, Budapest, Hungary). Each concentration of the activating substance studied was allowed to act for 15 min. Overall, 7 to 10 uteri were used per experiment.

It was found that H₂S alone caused a concentration-dependent inhibition of both the spontaneous and calcium-induced smooth muscle contraction (Figure 2A) with 1 mM of Na₂S yielding about 95% inhibition. Conversely, SNP alone had little effect over a wide range of concentrations (1-20 mM) for the spontaneous muscle contraction, and had a somewhat stronger inhibitory effect, IC₅₀ = 10 mM, for the Ca²⁺-induced contractions (Figure 2B). When mixed together, 1 mM of each SNP and H₂S caused an inhibitory effect with the same magnitude as the SNP alone for both the spontaneous and Ca²⁺-induced contractions (Figure 2C). Surprisingly, even a five-fold excess of H₂S over SNP proved to be ineffective for evoking a greater inhibition of the uterus contractions, which implies that any excess of H₂S is consumed under physiological (aerobic) conditions. Furthermore, these results clearly indicate that NO was not released by the reaction between SNP and H₂S.

### 4) Identification of the products from the reaction of SNP with H₂S

To identify the reaction products from SNP and H₂S, the corresponding reaction mixture was first analyzed using ultra-high resolution (UHR) ESI-TOF MS (maXis, Bruker Daltonics) with a low-temperature cryo-spray ionization (+4 °C) to prevent any potential temperature-induced decomposition of the products during ionization. The data analysis, mass convolution and spectral simulations were performed using the Data Analysis software provided by the manufacturer.

A negative mode mass spectrum of SNP revealed a molecular ion peak at m/z 238.9, which was assigned to Na[Fe(CN)₅(NO)]⁻, as also shown in Figure 3. However, upon addition of H₂S, all of the peaks for SNP disappeared, which indicates that the previously observed blue reaction product either does not ionize or fully decomposes under experimental conditions.

To gain further insight into the reaction mechanism and products, GC-MS and ¹⁴N NMR measurements were performed.

For GC-MS analysis, a 2 mM SNP solution at pH 7.4 was mixed with H₂S in a 1:1, 1:2 and 1:5 ratio, and the gas phase of the reaction mixture was analyzed (15 min after the reaction began) by gas chromatography with a triple-quadrupole mass spectrometer detector.

In particular, 2 mM SNP in 50 mM KPi pH 7.4 was degassed with argon and kept in dark glass vials sealed with PTFE septa until Na₂S was added to yield the respective final sulfide concentration. GC-MS analyses were performed on a Bruker GC 450 TQ MS 300. The gas chromatograph was equipped with a Varian VF-5m capillary column. Headspace gas samples 50 µL in volume were injected in the splitless mode. The following oven temperature program was used with helium as the carrier gas: ramped from 50 to 155 °C at a rate of 10 °C/min and then to 260 °C at a rate of 30 °C/min. The positive electron impact ionization mode was used. The detector multiplier voltage was set to 1400 V, and the detection was performed by selected ion monitoring of *mlz* 44 (corresponding to N₂O) and *m*/*z* 30 (corresponding to NO) with a dwell time of 50 ms and SIM scan width of 0.7 a.u. The areas under the peaks were determined using software provided by the manufacturer.

The results of the GC-MS analysis, presented in Figure 4 for the reaction mixture of SNP and H₂S in a ratio of 1:5, clearly show the characteristic mass spectrum for N₂O formation (*m*/*z* 44) (inset in Figure 4) (Wink, D.A. et al., 1996). The amount of N₂O formed over 15 min increased as the H₂S/SNP molar ratio increased and was highest in the reaction mixture containing a 5 fold excess of H₂S over SNP. No additional peak corresponding to NO was observed, which agrees with the above-described results obtained via amperometric detection of NO and H₂S and the above-described results from organ bath experiments using isolated rat uteri.

To further confirm this N₂O formation, an ¹⁴N NMR study was performed by collecting NMR spectra over 10 h. In particular, a 25 mM solution of SNP both with and without the addition of 25 mM Na₂S in 300 mM KPi pH 7.4 was placed in a 10 mm NMR tube, and the spectra were recorded using a Bruker 400 MHz spectrometer using the ¹⁵N signal of nitromethane as a reference. All of the spectra were recorded at 50.67 MHz, and 200 transients were collected with a 35° pulse width and a 5 s relaxation delay.

As shown in Figure 5A, the spectrum for 50 mM of SNP consists of two broad peaks with chemical shifts at -3.7 ppm and -90 ppm that were assigned to the nitrogen atoms from NO and CN- coordinated to paramagnetic Fe3⁺, respectively. The appearance of additional peaks was noticed 60 min after the addition of H₂S. Two of them, -142 ppm and -225 ppm, were assigned to N₂O (Figure 5B) (Strelenko, Y. A. et al., 1996). The N₂O signals increased over time and finally disappeared after 15 h. These results are in agreement with the GC-MS data. More importantly, the third signal in the NMR spectra was present in the more negative region (-355 ppm), which is characteristic of N-coordinated thiocyanate (SCN⁻) ligands (Figure 5A) (Mason, J. et al., 2002).

### 5) Mechanistic insights

To obtain additional mechanistic insight, the reaction between SNP and H₂S was followed in solution amperometrically using a selective H₂S electrode and via both time resolved UV/Vis spectroscopy and real-time FTIR spectroscopy.

When injected into a buffered solution, H₂S disappears over time due to its slow oxidation in ambient air. The presence of SNP in the buffered solutions (pseudo-first order conditions: 50 µM H₂S and 500 µM-10 mM SNP) led to the rapid removal of H₂S, which was determined to be concentration dependent, as also shown in Figure 6. An obvious decrease in the initial electrode signal with increasing SNP concentration indicates that the reaction is faster than the electrode response time. Therefore, the observed rates could have been underestimated. However, the linear dependence of the rate of H₂S removal on the SNP concentration can at least qualitatively imply a first-order reaction with respect to SNP.

To gain further mechanistic insight and detect possible intermediate species in the reaction pathway, rapid scan UV/Vis stopped-flow measurements were used. Kinetic data were obtained by recording the time-resolved UV/Vis spectra using a modified *µ*SFM-20 Bio-Logic stopped-flow module combined with a Huber CC90 cryostat and equipped with a J&M TIDAS high-speed diode array spectrometer containing both deuterium and tungsten lamps (200-1015 nm wavelength range) as described in Bisset, W.I.K. et al., 1981.

The reaction was performed under pseudo-first order conditions by applying an excess of H₂S. Unlike the studies described in the literature (Butler, A. R. et al., 1988; Rock, P. A. et al., 1965; Quiroga, S.L. et al., 2011), these measurements were conducted using a physiologically relevant pH 7.4 and under aerobic conditions.

The time-resolved spectra (Figure 7A) revealed the rapid formation (within the dead time of the stopped-flow device, about 3 ms) of an intermediate with an absorption maximum at 535 nm for the first reaction step (Figure 7B). This absorption maximum is characteristic of the RSNOs coordinated to iron(II) observed as the pink product of SNP reactions with other thiols (Butler, A. R. et al., 1988; Grossi, L. et al., 2005; Johnson, M. D. et al., 1983). This intermediate can be defined as the HSNO-iron adduct, [(CN)₅FeN(O)SH]³⁻, assuming the pKa value for the coordinated HSNO is 10.5, as previously reported (Quiroga, S.L. et al., 2011). This reaction showed a dependence on the H₂S concentration. The intensity of the UV/Vis absorption band at about 535 nm was strongly dependent on the H₂S concentration, which suggests that the [(CN)₅FeN(O)SH]³⁻ adduct formation is a reversible process in accordance with previous observations (Quiroga, S.L. et al., 2011).

The fate of this adduct was then examined, which proved to be short-lived at pH 7.4, as it rapidly transformed into a final blue species with an absorbance maximum at 575 nm and a broad UV/Vis band (shoulder) at about 390 nm (Figure 7B-C). Additional transient species appeared on the transformation pathway (which were not previously observed) that exhibited absorbance maximum at about 720 nm and 440 nm (spectra at the bottom of Figure 7A).

Although a species with an absorbance maximum at 575 nm was ascribed to [(CN)₅FeN(O)S]⁴⁻ (deprotonated form of the initial [(CN)₅FeN(O)SH]³⁻ adduct) in the recent literature (Quiroga, S.L. et al., 2011), it should be noted that some previous work (Swinehart, J. H., 1967) on light-induced SNP reactions with SCN⁻ reported the exact same spectral features (absorption maximum at ∼575 nm). This finding implies a thiocyanate coordination occurred as a result of the photo-induced NO release (Swinehart, J. H., 1967). In general, the resulting blue-violet color and an absorbance maximum at 575 nm are typical of Fe(II)-thiocyanato species (Roncaroli, F. et al., 2002), especially those with more SCN- ligands (between 4 and 6) that can even be maintained under aerobic conditions (also while starting from the Fe(III) species) (Kratochvil, B. et al., 1970). The same product (Figure 8) could be obtained by mixing [Fe^{II}(CN)₅(H₂O)]³⁻ with an excess of SCN⁻. Furthermore, our ¹⁴N NMR studies contained a signal at -355 ppm that is characteristic of N-coordinated thiocyanate (Figure 5A). The consistent, continuous shifting of the 535 nm band towards longer wavelengths during the transformation of the initial [(CN)₅FeN(O)SH]³⁻ adduct (Figure 7B-C) could imply a consecutive increase in the number of coordinated SCN- ions on the Fe(II) center. Similar behavior has been reported for the binding of thiocyanate to another type of iron center (Sarauli, D. et al., 2007).

The 720 nm band for the above mentioned transient species is characteristic of a mixed-valent cyano-bridged complex (a Prussian blue type compound) that could be independently obtained either from the reaction of aqua [Fe^{II}(CN)₅₋ₓ(SCN)ₓH₂O)]³⁻ complexes in the presence of oxygen or upon addition of the corresponding Fe(III) complexes (Roncaroli, F. et al., 2002). The UV/Vis band at approximately 440 nm can be attributed to transient [(CN)₅Fe(HNO)]³⁻ (spectra at the bottom of Figure 7A) (Montenegro, A.C. et al., 2009).

The transformation of the initial [(CN)₅FeN(O)SH]³⁻ adduct into the final thiocyanato species was faster with increasing H₂S and oxygen concentrations. However, the concentration dependence proved to be rather complicated because of a) the cross-interaction between H₂S (i.e., HSₓ⁻HS₍ₓ₊₁₎^{·2-}.) and oxygen, b) the involvement of oxygen in the generation of the transient mixed-valent Fe(II)/Fe(III) species and c) the involvement of the HSₓ⁻/HS₍ₓ₊₁₎^{·2-} species in the transformation of CN⁻ to SCN⁻ (Luthy, R. G. et al., 1979). The detailed quantification of the H₂S and O₂ concentration-dependent kinetic behavior would require a separate study.

The proposed mechanism is in good agreement with the solution in real-time FTIR measurements, which monitored the characteristic vibrations of SNP in the 1700 - 2500 cm⁻¹ region. Namely, the IR spectrum of SNP in the solution contained three particularly intense bands at 1924, 2143 and 2258 cm⁻¹ assigned to the NO, radial CN⁻, and axial CN- stretching frequencies, respectively, as described previously (Swinehart, J. H., 1967). The addition of H₂S (in fourfold excess) caused an immediate change that led to the disappearance of the NO (1924 cm⁻¹) and radial CN- (2144 cm⁻¹) stretching bands with a concomitant formation of two new peaks at 2056 and 2091 cm⁻¹ (Figure 9A). While the first of these peaks reached a maximum after a few min and remained in solution for over a few hours (Figure 9B), the latter completely disappeared after 2 min (Figure 9C). Based on the overall results, it is proposed that the first band corresponds to the coordinated thiocyanate, as reported previously (Ivanovic-Burmazovic, I. et al., 2002), while the latter could originate from the formation of an intermediate species with an absorbance maximum at 720 nm. These results imply a rhodanese-like activity whenever [Fe(CN)₅₋ₓ(SCN)ₓ(H₂O)]³⁻ with x ≤5 is formed, while HNO is released concomitantly.

To further prove that thiocyanate (SCN⁻) formation occurs, 1 mM SNP samples treated with a 2, 5 and 10 fold excess of H₂S were analyzed by GC-MS via an extractive alkylation, with pentafluorobenzyl bromide (PFB-Br) as the alkylating agent and tetrabutylammonium sulfate (TBA) as the phase-transfer catalyst (Paul, B.D. et al., 2006). 2,5-Dibromotoluene (DBT) was used as an internal standard for the quantitation of SCN⁻. The products were analyzed by gas chromatography-mass spectrometry on a Bruker GC 450 TQ MS 300.

The results of the GC-MS detection of SCN- are shown in Figure 10 and in Table 1 below. The data in Table 1 demonstrate that SCN- could be clearly detected in all of the samples with a yield dependent on the H₂S concentration. The relatively low amounts of SCN- detected are in agreement with the fact that only free thiocyanate anions that transfer into the ethylacetate phase can be detected. The total amount of SCN- increased significantly when the reaction mixtures were left overnight, which indicates that the labile Fe(II) complex decomposed and formed free SCN⁻ in solution.

**Table 1: Detection of SCN- formed during the reaction of 1 mM SNP with 2, 5 or 10 mM H₂S. The samples were analyzed both 5 min and 24 h after the reaction had started. SCN⁻ was detected using an extractive alkylation protocol involving the GC-MS detection of the final pentafluorobenzyl thiocyanate.**

| **SNP: H₂S ratio** | **5 min** | **24 h** |
|---|---|---|
| **1:2** | 200 ± 5 µM | 315 ± 12 µM |
| **1:5** | 467 ± 7 µM | 856 ± 11 µM |
| **1:10** | 511 ± 7 µM | 1038 ± 8 µM |

These results demonstrate that a nitroprusside salt and a sulfide salt, such as SNP and Na₂S, react under physiological conditions to provide HNO, without any intermediate formation of NO. Moreover, toxic cyanide is not released from the nitroprusside complex but is rather converted into innocuous thiocyanate.

### Example 2: Generation of HNO from SNP and H₂S in a biological milieu and physiological consequences of this reaction mechanism

### 1) Intracellular detection of HNO formed from the SNP/H₂S reaction mixture

It was further sought to confirm the formation of HNO from the SNP and H₂S mixture in a biological milieu. To accomplish this goal, a recently developed Cu²⁺-based fluorescent sensor, CuBOT1, was used for the intracellular HNO detection (Rosenthal, J. et al., 2009).

Human umbilical vein endothelial cells (HUVEC, passage 2-3) were obtained from PromoCell GmbH (Heidelberg, Germany) and cultured in 35 mm µ-dishes (ibidi, Martinsried, Germany) using a commercially available cell growth medium (PromoCell GmbH) at 37 °C and 5 % CO₂. For NO detection, the cells were preloaded for 10 min with 10 µM 4-amino-5-methylamino-2',7'-difluorofluorescein diacetate (DAF-FM-DA). For HNO detection, the HUVECs were incubated for 20 min with 10 µM CuBOT1 (Rosenthal, J. et al., 2009; Filipovic, M.R. et al., 2012, b). For both NO detection and HNO detection, the HUVECs were then washed three times and treated with 100 µM SNP, 100 µM H₂S or the mixture of both for 15 min. Fluorescence microscopy was performed using an inverted microscope (Axiovert 40 CLF, Carl Zeiss) equipped with green fluorescent filters and an AxioCam. The images were processed using ImageJ software.

The intracellular formation of the fluorescent Cu⁺BOT1 was visualized by fluorescence microscopy. Neither H₂S alone nor SNP alone caused any detectable change in the fluorescent intensity; however, the combination of these two molecules increased the fluorescence, as shown in Figure 11. For cells previously loaded with the NO fluorescent sensor DAF-FM-DA, treatment with the SNP and H₂S reaction mixture caused no significant changes in the fluorescence intensity relative to the control (data not shown). These results are in good agreement with the further data presented herein and confirm the observation that coordinated NO is not released from SNP but is efficiently reduced to HNO.

### 2) Ex-vivo release of CGRP from isolated hearts induced by the SNP/H₂S reaction mixture

The primary proposed biological marker for HNO formation is the increased release of calcitonin gene-related peptide (CGRP), a potent vasodilator (Paolocci, N. et al., 2001). CGRP is a neuropeptide for chemosensory primary afferent nerve fibers located in the epicardial surface of the heart.

The release of CGRP from the isolated heart of C57BL/6 mice was measured as reported in Strecker, T. et al., 2006. Briefly, the mice were suffocated in a pure CO₂ atmosphere and the complete heart was excised by cutting the central blood vessels. The procedures were approved by the animal protection authorities (of the "Regierung von Mittelfranken", Ansbach, Germany). The hearts were passed through a series of synthetic interstitial fluid incubations both with and without the addition of the test compounds (H₂S alone, SNP alone, or the combination of H₂S and SNP) and spent 5 min in each test tube at 37 °C (details are indicated in Figure 12). The CGRP concentration was measured via enzyme immunoassay (Bertin Pharma, Montigny, France).

Using this experimental model, it was shown that 1 mM of H₂S had no effect on CGRP release, while 500 µM SNP induced a small increase that could have originated from the reaction of SNP with intracellular H₂S. The combination of both H₂S and SNP, however, significantly increased the CGRP release (p = 0.028, n = 6, Wilcoxon matched pairs test), as also shown in Figure 13.

These results strongly confirm that HNO is formed from the combination of a nitroprusside salt with a sulfide salt, resulting in the release of the potent vasodilator CGRP from the heart. This indicates that the combination of a nitroprusside salt or solvate with a sulfide salt or solvate according to the present invention, including in particular the combination of SNP with Na₂S, is highly effective in the medical intervention of cardiovascular diseases/disorders.

### 3) Reaction mechanism of SNP with H₂S and its physiological consequences

Despite the long interest in the reactions of H₂S with SNP since 1850 (Sidgwick, N.V., 1950), the reaction mechanism has eluded researchers due primarily to methodological limitations. The present invention provides the first study to combine chemical tools with pharmacological/physiological experiments at a physiological pH and under aerobic conditions to elucidate the reaction between SNP and H₂S. Based on the results presented herein, the overall reaction mechanism can be depicted as shown in Scheme 2 below. 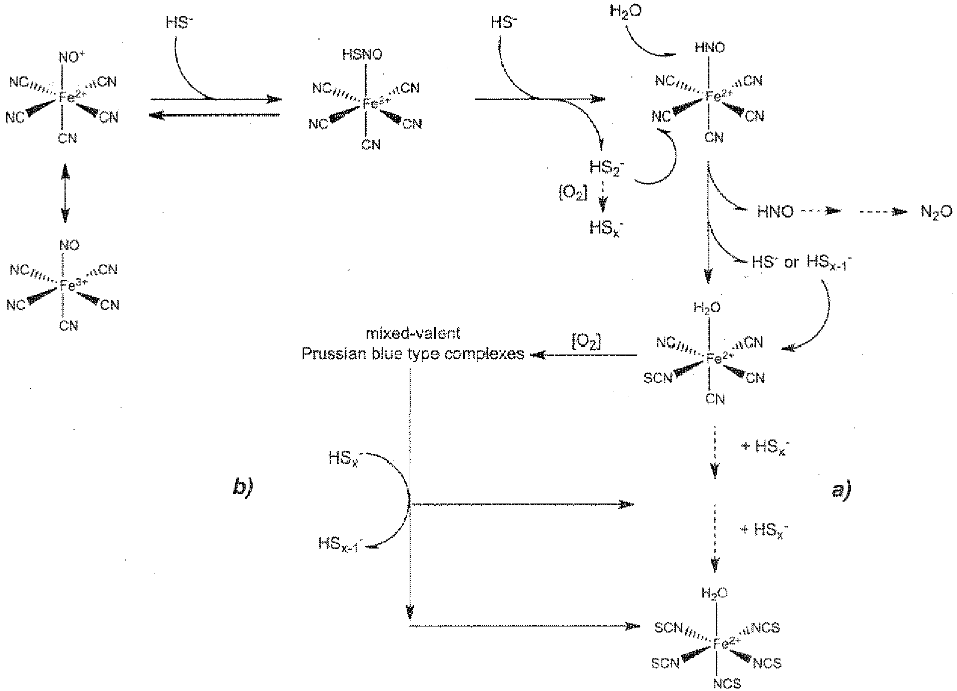
- **Scheme 2:**: Mechanistic depiction of the reaction between SNP and H₂S at pH 7.4 under aerobic conditions. SNP reacts very rapidly with H₂S to form an intermediate [(CN)₅FeN(O)SH]³⁻. In the second reaction step, H₂S reduces the coordinated HSNO/SNO⁻ and initially becomes oxidized to disulfide, which can be further oxidized to polysulfides as the reaction progresses. As a result of this step, the intermediate [(CN)₅Fe(HNO)]³⁻ is formed, which is unreactive towards H₂S but does react with polysulfides to form a thiocyanate adduct upon the elimination of HNO. HNO can dimerize to yield N₂O, while the other coordinated cyanides are consecutively transformed into thiocyanates (main reaction path, *a*). Oxygen can oxidize the iron(Il) center and create the mixed-valent bridged complexes known as Prussian blue, which are short-lived and further react with polysulfides to give the final thiocyanate products as well (minor reaction route, *b*).

SNP reacts very rapidly with H₂S to form the intermediate [(CN)₅FeN(O)SH]³⁻, which is further transformed into the thiocyanate product(s). During the second reaction step, H₂S plays a catalytic role. Upon reducing the coordinated HSNO/SNO⁻, it initially oxidizes to disulfide, which can oxidize to form the polysulfides used in the reaction progress. As a result of this step, a [(CN)₅Fe(HNO)]³⁻ intermediate formed, as indicated by the time-resolved UV/Vis spectral analysis (440 nm band, see spectra at the bottom of Figure 7A). The reaction of thiols (RSH) and H₂S with S-nitrosothiols (R'SNO) to generate HNO and disulfides (RSSR') is a well-established process (Arnelle, D.R. et al., 1995; Filipovic, M.R. et al., 2012, b). [(CN)₅Fe(HNO)]³⁻ is prepared by following the literature protocol (Montenegro, A.C. et al., 2009) using SNP and two equivalents of dithionite. This complex proved to be stable toward H₂S (Figure 14); however, it could react with S-S bond containing compounds and convert the CN- into SCN⁻ as for any cyanide species (Luthy, R. G. et al., 1979). It has been shown through independent experiments that [(CN)₅Fe(HNO)]³⁻ reacts with polysulfides to generate this thiocyanate product with an absorption maxima of about 575 nm and a shoulder at about 390 nm (Figure 14). This process most likely labilizes HNO, which can be released and/or further dimerize into N₂O.

The production of both N₂O (Figures 4 and 5) and HNO (Figure 11) has thus been demonstrated, whereas no evidence for the release of NO could be found. Upon release of HNO, the aqua complex [(CN)₄(SCN)Fe(H₂)]³⁻ is formed and is a source of mixed-valent cyano-bridged adducts due to the inevitable existence of some Fe(III) species under aerobic conditions. Both of these adducts, as well as any other cyanide containing species, further react with the disulfide/polysulfides generated during the CN- to SCN- conversion of the ligands. Although a thorough kinetic analysis would require a separate study, the kinetic traces, which correspond to the formation of the final product, are indicative of an induction period (Figure 7D). This period is related to the catalytic nature of the process and the preformation of mixed-valent cyano-bridged adducts. Therefore, the duration of the induction period also depends on the H₂S and O₂ concentrations with shorter durations corresponding to an increased H₂S concentration and longer durations to an increased O₂ concentration. Overall, all these data are indicative of the operation of a novel, previously undescribed mechanism (Scheme 2) with physiologically important features: the release of HNO at a physiological pH of 7.4 and the conversion of cyanides into thiocyanates with the latter generally being catalized *in vivo* by rhodaneses (Mueller, E.G., 2006; Cipollone, R. et al., 2007). Rhodanese is the common name for a thiosulfate:cyanide sulfurtransferase class of enzymes that use thiosulfate to catalyze the detoxification of cyanide (Cipollone, R. et al., 2007). This enzyme is found in mitochondria and the mechanism of its action is similar to what has presently been observed for the reaction of SNP with H₂S: persulfides (formed during the reaction of thiosulfate with the free protein thiols in the enzyme) react with cyanide to form thiocyanate (Cipollone, R. et al., 2007).

The formation of HNO is important because it is considered to be a very promising therapeutic agent for the treatment of cardiovascular diseases (Flores-Santana, W. et al., 2011; Irvine, J.C. et al., 2008). As a redox congener of NO, HNO reacts in the cell orthogonally to NO. It stimulates CGRP release (Wink, D.A. et al., 2003), among other things, and accounts for the positive inotropic heart effects observed when an HNO donor was used (Paolocci, N. et al., 2001; Paolocci, N. et al., 2007). It has been shown that, unlike NO donors, HNO donors do not induce a nitrate-tolerance (Bullen, M.L. et al., 2011). Because the intracellular production of HNO is still a matter of debate, with evidence showing its production from NO via NO synthase (Schmidt, H.H. et al., 1996), cytochrome c (Sharpe, M.A. et al., 1998) and manganese superoxide dismutase (MnSOD) (Filipovic, M.R. et al., 2007) or from hydroxylamine by heme-containing proteins (Donzelli, S. et al., 2008), the synthesis of donors that could release HNO under physiological conditions is one of the current pharmacological goals (DuMond, J.F. et al., 2011; Shoman, M.E. et al., 2011; Keefer, L.K., 2011).

SNP is still used in acute hypertensive crises to regulate blood pressure. However, it must be combined with thiosulfate to minimize the toxic effects of cyanide through natural rhodanese activity (Bisset, W.I.K. et al., 1981; Pasch, T. et al., 1983). The results provided herein offer a new perspective on the clinical application of SNP. The combination of SNP with a sulfide salt as an H₂S donor, which causes the HNO-induced release of CGRP and transforms toxic cyanide into thiocyanate, provides a new, more effective and less toxic therapeutic alternative to the combination of SNP with thiosulfate. Furthermore, by accounting for the recently revised plasma levels of H₂S (in the micromolar range) (Shen, X. et al., 2011), it can be speculated that part of the vasodilatory effects of SNP could be due to reactions with circulatory H₂S via a rhodanese-like mechanism.

### REFERENCES

Ali, M.Y. et al., Br. J. Pharmacol. 2006, 149, 625-634.
Appiah, I. et al., BrJ. Pharm. 2010, 158, 1932-1941.
Arnelle, D.R. et al., Arch. Biochem. Biophys. 1995, 318, 279-285.
Bisset, W.I.K. et al., Br. J. Anaesth. 1981, 53, 1015-1018.
Brand, E. et al., J. Biol. Chem. 1930, 86, 315-331.
Brauer, G. (Ed.), Handbook of Preparative Inorganic Chemistry, Vol. 1., 2nd Ed., 1963, Academic Press, NY., p 1768.
Bullen, M.L. et al., Antioxid. Redox Signal. 2011, 14, 1675-1686.
Butler, A. R. et al., Polyhedron. 1988, 7, 1197-1202.
Butler, A. R. et al., Chem Rev. 2002, 102, 1155-1165.
Caliendo G. et al., J Med Chem. 2010, 53(17), 6275-6286.
Cipollone, R. et al., IUBMB Life, 2007, 59, 51-59.
Demirkoprulu, N. et al., Eur. J. Pharmacol. 2005, 517, 240-245.
Donzelli, S. et al., Free Radic. Biol. Med. 2008, 45, 578-584.
DuMond, J.F. et al., Antioxid. Redox Signal. 2011, 14, 1637-1648. Filipovic, M.R. et al., Free Radic. Res. 2007, 41, 62.
Filipovic, M. R. et al., Chem. Eur. J., 2012, 18, 13538-13540. (a).
Filipovic, M.R. et al., J. Am. Chem. Soc. 2012, 134, 12016-12027. (b).
Filipovic, M.R. et al., *Biochem J.* **2012,** *441*, 609-621. (c).
Flores-Santana, W. et al., Antioxid. Redox. Signal. 2011, 14, 1659-1674.
Grossi, L. et al., J. Med. Chem. 2005, 48, 2622-2626.
Hennan, J. K. et al., *Br. J. Pharmacol.* **1998,** *123*, 959-967.
Hosoki, R. et al., Biochem Biophys Res Commun. 1997, 237(3), 527-531.
Hughes M.N. et al., Free Radic Biol Med. 2009, 47(10), 1346-1353.
Hyde, F.S., J Am Chem Soc. 1897, 19(1), 23-24.
Irvine, J.C. et al., Trends Pharmacol. Sci. 2008, 29, 601-608,
Ivanovic-Burmazovic, I. et al., Inorg Chem. 2002, 41, 5150-5161.
Johnson, M. D. et al., Inorg. Chem. 1983, 23, 231-235.
Kashfi K. et al., Biochem Pharmacol. 2012 (in press), doi:10.1016/j.bcp.2012.10.019
Keefer, L. K., ACS Chem. Biol. 2011, 6, 1147-1155.
Kemsley, J., Chemical & Engineering News. 2012, 90(28), 5
Kratochvil, B. et al., Anal. Chem. 1970, 42, 43-46.
Li, L. et al., Pharmacol. Therap. 2009, 123, 386-400.
Li, L. et al., Ann. Rev. Pharmacol. Toxicol. 2011, 51, 169-187.
Luthy, R. G. et al., Environ. Sci. Technol. 1979, 13, 1481-1487.
Mason, J. et al., Chem Rev. 2002, 102, 913-934.
Montenegro, A.C. et al., Angew. Chem. 2009, 48, 4213-4216.
Mueller, E.G. et al., Nat. Chem. Biol. 2006, 2, 185-194
Mustafa, A.K. et al., Circ. Res. 2011, 109, 1259-1268.
Paolocci, N. et al., Proc. Natl. Acad. Sci. USA. 2001, 98,10463-10468.
Paolocci, N. et al., Pharmacol. Ther. 2007, 113, 442-458.
Pasch, T. et al., J. Cardiovasc. Pharmacol. 1983, 5, 77-86.
Paul, B. D. et al., J. Anal. Toxicol. 2006, 30, 511-515.
Quiroga, S.L. et al., Chemistry. Eur. J. 2011, 17, 4145-4156.
Rock, P. A. et al., Inorg. Chem. 1965, 5, 1078-1079.
Roncaroli, F. et al., Inorg. Chem. 2002, 41, 5417-5425.
Roncaroli, F. et al., Coord Chem Rev. 2007, 251, 1903-1930.
Rosenthal, J et al., J. Am. Chem. Soc. 2009, 132, 5536-5537.
Sarauli, D. et al., Inorg. Chem. 2007, 46, 7848-7860.
Schmidt, H.H. et al., Proc. Natl. Acad. Sci. U.5.A. 1996, 93, 14492-11197.
Sharpe, M.A. et al., Biochem. J. 1998, 332, 9-19.
Shen, X. et al., Free Radic. Biol. Med. 2011, 50, 1021-1031.
Shoman, M. E. et al., J. Med. Chem. 2011, 54, 1059-1070.
Sidgwick, N.V. The chemical Elements and Their Compounds II, Oxford University Press, London, 1950; p 1345.
Stamler, J.S.; Feelisch, M. Preparation and Detection of S-Nitrosothiols. In: Methods in Nitric Oxide Research. Feelisch, M., Stamler, J. S., Eds, John Wiley & Sons, Ltd, Chichester, England, 1996; pp 521-539.
Strecker, T. et al., Neuropeptides. 2006, 46, 107-113.
Strelenko, Y. A. et al., J. Mol. Struct. 1996, 378, 61-65.
Swinehart, J. H., Coord. Chem. Rev. 1967, 2, 385-402.
Szabo, C., Nature Rev. Drug Disc. 2007, 6, 917-935.
Szacilowski, K. et al., New J. Chem. 2002, 26, 1495-1502.
Wallace, J.L., Trends Pharmacol Sci. 2007, 28(10), 501-505.
Wang, Y.F. et al., Chin Med J (Engl). 2008, 121(5), 420-423.
White S.A. et al., Anaesthesia. 1997, 52(5), 422-427.
Whiteman, M. et al., Biochem. Biophys. Res. Commun. 2006, 343, 303-310.
Wink, D.A.; Feelisch, M. Formaton and Detection of Nitroxyl and Nitrous Oxide. In: Methods in Nitric Oxide Research. Feelisch, M., Stamler, J. S., Eds, John Wiley & Sons, Ltd, Chichester, England, 1996; pp 403-412.
Wink, D.A. et al., Am. J. Physiol. Heart Circ. Physiol. 2003, 285, H2264-2276.
Yang, G. et al., Science. 2008, 322, 587-559.
Yong, Q.C. et al., Cardiovasc Res. 2010, 88, 482-491.
Yong, Q.C. et al., Antioxid. Redox. Signal. 2011, 14, 2081-2091*.*
Zhao, Y. et al., J Am Chem Soc. 2011, 133(1), 15-17.

## Claims

1. A nitroprusside salt or a solvate thereof for use in the treatment or prevention of a cardiovascular disease/disorder, wherein the nitroprusside salt or solvate is to be administered in combination with a sulfide salt or a solvate thereof.

2. A sulfide salt or a solvate thereof for use in the treatment or prevention of a cardiovascular disease/disorder, wherein the sulfide salt or solvate is to be administered in combination with a nitroprusside salt or a solvate thereof.

3. A pharmaceutical composition for use in the treatment or prevention of a cardiovascular disease/disorder, the pharmaceutical composition comprising a nitroprusside salt or a solvate thereof in combination with a sulfide salt or a solvate thereof and optionally a pharmaceutically acceptable excipient.

4. A method of treating or preventing a cardiovascular disease/disorder, the method comprising the administration of a nitroprusside salt or a solvate thereof in combination with a sulfide salt or a solvate thereof to a subject in need thereof.

5. The nitroprusside salt or solvate for use according to claim 1 or the sulfide salt or solvate for use according to claim 2 or the method of claim 4, wherein the nitroprusside salt or solvate and the sulfide salt or solvate are to be administered simultaneously or sequentially.

6. The nitroprusside salt or solvate for use according to claim 1 or 5 or the sulfide salt or solvate for use according to claim 2 or 5 or the pharmaceutical composition for use according to claim 3 or the method of claim 4 or 5, wherein the nitroprusside salt is sodium nitroprusside.

7. The nitroprusside salt or solvate for use according to claim 1, 5 or 6 or the sulfide salt or solvate for use according to claim 2, 5 or 6 or the pharmaceutical composition for use according to claim 3 or 6 or the method of any of claims 4 to 6, wherein the sulfide salt or solvate is an alkali hydrogensulfide salt, a dialkali sulfide salt, an alkaline earth metal sulfide salt, or a solvate thereof.

8. The nitroprusside salt or solvate for use according to any of claims 1 or 5 to 7 or the sulfide salt or solvate for use according to any of claims 2 or 5 to 7 or the pharmaceutical composition for use according to claim 3, 6 or 7 or the method of any of claims 4 to 7, wherein the sulfide salt or solvate is Na₂S.

9. The nitroprusside salt or solvate for use according to any of claims 1 or 5 to 8 or the sulfide salt or solvate for use according to any of claims 2 or 5 to 8 or the pharmaceutical composition for use according to any of claims 3 or 6 to 8 or the method of any of claims 4 to 8, wherein the sulfide salt or solvate is to be used in an about 5-fold to about 6-fold molar excess with respect to the amount of the nitroprusside salt or solvate.

10. The nitroprusside salt or solvate for use according to any of claims 1 or 5 to 9 or the sulfide salt or solvate for use according to any of claims 2 or 5 to 9 or the pharmaceutical composition for use according to any of claims 3 or 6 to 9 or the method of any of claims 4 to 9, wherein the cardiovascular disease/disorder is selected from heart failure, heart attack or myocardial infarction, hypertension, coronary obstruction, coronary artery disease, angina pectoris, ischemic cardiomyopathy and/or infarction, pulmonary congestion, pulmonary edema, cardiac fibrosis, valvular heart disease, pericardial disease, circulatory congestive state, peripheral edema, ascites, myocarditis, Chagas disease, ventricular hypertrophy, or heart valve disease.

11. The nitroprusside salt or solvate for use according to any of claims 1 or 5 to 10 or the sulfide salt or solvate for use according to any of claims 2 or 5 to 10 or the pharmaceutical composition for use according to any of claims 3 or 6 to 10 or the method of any of claims 4 to 10, wherein the cardiovascular disease/disorder is selected from congestive heart failure, acute congestive heart failure, acute decompensated heart failure, diastolic heart failure, or cardiac asthma.

12. The nitroprusside salt or solvate for use according to any of claims 1 or 5 to 10 or the sulfide salt or solvate for use according to any of claims 2 or 5 to 10 or the pharmaceutical composition for use according to any of claims 3 or 6 to 10 or the method of any of claims 4 to 10, wherein the cardiovascular disease/disorder is selected from hypertensive heart disease, hypertensive nephropathy, essential hypertension, secondary hypertension, renovascular hypertension, pulmonary hypertension, malignant hypertension, benign hypertension, systolic hypertension, white coat hypertension, or acute hypertension.

13. The nitroprusside salt or solvate for use according to any of claims 1 or 5 to 12 or the sulfide salt or solvate for use according to any of claims 2 or 5 to 12 or the pharmaceutical composition for use according to any of claims 3 or 6 to 12 or the method of any of claims 4 to 12, wherein the compound or the pharmaceutical composition is to be administered in combination with a further pharmaceutically active agent.

14. The nitroprusside salt or solvate for use according to claim 13 or the sulfide salt or solvate for use according to claim 13 or the pharmaceutical composition for use according to claim 13 or the method of claim 13, wherein the further pharmaceutically active agent is selected from glyceryl trinitrate, isosorbide dinitrate, isosorbide mononitrate, linsidomine, molsidomine, pentaerythritol tetranitrate, propatylnitrate, tenitramine, trolnitrate, flosequinan, itramin tosilate, prenylamine, oxyfedrine, benziodarone, carbocromen, hexobendine, etafenone, heptaminol, imolamine, dilazep, trapidil, molsidomine, efloxate, cinepazet, cloridarol, nicorandil, nesiritide, gapicomine, pimobendan, levosimendan, berberine, levosimendan, omecamtiv, dopamine, dobutamine, dopexamine, epinephrine, adrenaline, isoprenaline, isoproterenol, norepinephrine, noradrenaline, digoxin, digitalis, prostaglandins, enoximone, milrinone, amrinone, theophylline, glucagon, insulin, acetazolamide, furosemide, bumetanide, etacrynic acid, etozoline, muzolimine, piretanide, tienilic acid, torasemide, hydrochlorothiazide, bendroflumethiazide, hydroflumethiazide, chlorothiazide, polythiazide, trichlormethiazide, cyclopenthiazide, methyclothiazide, cyclothiazide, mebutizide, quinethazone, clopamide, chlortalidone, mefruside, clofenamide, metolazone, meticrane, xipamide, indapamide, clorexolone, fenquizone, amiloride, triamterene, benzamil, spironolactone, eplerenone, potassium canrenoate, canrenone, mannitol, urea, conivaptan, mozavaptan, satavaptan, tolvaptan, demeclocycline, mersalyl, meralluride, theobromine, cicletanine, alprenolol, bopindolol, bupranolol, carteolol, cloranolol, mepindolol, nadolol, oxprenolol, penbutolol, pindolol, propranolol, sotalol, tertatolol, timolol, acebutolol, atenolol, betaxolol, bevantolol, bisoprolol, celiprolol, epanolol, esmolol, nebivolol, metoprolol, practolol, S-atenolol, talinolol, carvedilol, labetalol, or butaxamine.

15. The method of any of claims 4 to 14, wherein the subject is a human or a non-human animal.
